# EUROPÄISCHE PATENTSCHRIFT

(11) **EP 0 860 046 B1**
(45) Veröffentlichungstag und Bekanntmachung des Hinweises auf die Patenterteilung: **26.02.2003**
(21) Anmeldenummer: 96930012.8
(22) Anmeldetag: 26.09.1996
(51) Int. Cl.: H02K 7/09, A61M 1/10, F04D 13/06, F16C 39/06

(54) **ROTATIONSPUMPE**
ROTARY PUMP
POMPE ROTATIVE

(43) Veröffentlichungstag der Anmeldung: 26.08.1998
(73) Patentinhaber: Levitronix LLC, Waltham, MA 02451 (US)
(72) Erfinder: SCHÖB, Reto, CH-8604 Volketswil (CH); HUGEL, Jörg, CH-8008 Zürich (CH)
(74) Vertreter: Heinen, Detlef
(86) Internationale Anmeldenummer: CH9600335
(87) Internationale Veröffentlichungsnummer: WO98011650

(56) Entgegenhaltungen:
- EP-A- 0 467 234
- DE-A- 2 406 790
- FR-A- 2 681 384
- US-A- 4 779 614
- US-A- 5 112 200
- US-A- 5 385 581
- US-A- 5 470 208
- US-A- 5 484 266
- ESAO, 1. - 3.September 1982, BRUSSELS, BELGIUM, Seiten 215-218, XP002029773 G BRAMM ET AL: "Axial Centrifugal Blood Pump with Electromagnetically Suspended Rotor"

## Beschreibung

Die Erfindung betrifft eine Rotationspumpe gemäss dem Oberbegriff von Anspruch 1 (vgl. doku. US-A-4 779 614).

Bekannte Rotationspumpen, zum Beispiel Axialpumpen oder Zentrifugalpumpen, weisen einen durch mechanische Lagervorrichtungen drehbar gelagerten Rotor auf. Derartige Rotationspumpen weisen den Nachteil auf, dass das geförderte Fluid durch Schmiermittel oder mechanische Abriebe der Lagervorrichtungen oder des Rotors verunreinigt wird, sodass derartige Pumpen nicht geeignet sind Fluide zu fördern, die nicht verunreinigt werden dürfen. Solche Fluide sind zum Beispiel Reinstwasser oder Blut. Zudem sind derartige Rotationspumpe nicht geeignet aggressive Flüssigkeiten zu fördern, da die Lagervorrichtungen in kurzer Zeit zerstört würden.

Es ist Aufgabe der vorliegenden Erfindung eine wirtschaftlich vorteilhaftere Rotationspumpe vorzuschlagen.

Diese Aufgabe wird gelöst mit einer Rotationspumpe gemäss den Merkmalen von Anspruch 1. Die Unteransprüche 2 bis 20 beziehen sich auf weitere, vorteilhalfte Ausgestaltungen der Rotationspumpe.

Die Aufgabe wird gelöst durch eine Rotationspumpe mit einem berührungslos durch magnetisch wirkende Kräfte innerhalb eines Gehäuses der Rotationspumpe gelagerten und antreibbaren Rotor. Ein Vorteil der Erfindung ist darin zu sehen, dass das Gehäuse eine hermetische Trennung zwischen den ausserhalb des Gehäuses angeordneten, ansteuerbaren Elektromagneten und dem innerhalb des Gehäuses fliessenden Fluides erlaubt. Das Gehäuse weist keinerlei Durchbrüche auf. Zudem ist der Rotor berührungslos innerhalb des Gehäuses gelagert, sodass weder schmierende Mittel erforderlich sind noch ein Abrieb durch Lagervorrichtungen entsteht.

Die Erfindung wird insbesondere durch eine Rotationspumpe umfassend ein Gehäuse und ein innerhalb des Gehäuses angeordnetes Pumpenrad mit einer Drehachse gelöst, bei welcher das Pumpenrad ein passives, magnetisch wirksames Rotorteil umfasst und das Gehäuse von einem elektrische Spulen und Zähne aufweisenden Stator umgeben ist, welche derart angeordnet, ausgestaltet und ansteuerbar sind, dass der Stator und der Rotorteil als ein lagerloser Motor zusammenwirken.

Durch die Verwendung eines lagerlosen Motors ist der Rotorteil bezüglich dreier Freiheitsgrade aktiv ansteuerbar und sowohl die Lage des Rotorteils in einer senkrecht zur Drehachse verlaufenden Ebene in x- und y-Richtung als auch ein in Umfangsrichtung auf das Rotorteil bewirkte Drehmoment durch eine entsprechende Ansteuerung der elektrischen Spulen vorgebbar.

Der Rotorteil und die Zähne des Stators sind derart geometrisch gegenseitig angepasst ausgestaltet und zueinander angeordnet, dass der Rotorteil bezüglich dreier weiterer, nicht aktiv ansteuerbarer Freiheitsgrade, durch passiv wirkende Reluktanzkräfte im Stator haltbar ist, um den Rotor innerhalb des Gehäuses antreibbar und stabil berührungslos schwebend anzuordnen. Ein Vorteil dieser Anordnung ist darin zu sehen, dass die Lage des Rotors bezüglich einer senkrecht zur Drehachse verlaufenden Ebene aktiv ansteuerbar ist, dass ein ansteuerbares Drehmoment auf den Rotor bewirkbar ist, und dass die Lage des Rotors bezüglich der weiteren drei Freiheitsgrade durch passiv wirkende magnetische Kräfte in einer stabilen Lage im Stator gehalten ist.

Die erfindungsgemässe Rotationspumpe ist vorzugsweise als eine Axialpumpe oder eine Zentrifugalpumpe ausgebildet, wobei der Rotor entsprechend als ein Axialrad einer Axialpumpe oder als ein Zentrifugalrad einer Zentrifugalpumpe ausgebildet ist.

Der Rotor, als Axialrad ausgebildet, erzeugt auf das geförderte Fluid einen in axialer Richtung wirkenden Schub. Der Rotor, als Zentrifugalrad ausgebildet erfährt während dem Pumpbetrieb ebenfalls eine in axialer Richtung wirkende Kraft. Der magnetisch wirksame Rotorteil, Bestandteil des Axialrades oder des Zentrifugalrades, ist in Richtung der Drehachse bzw. in axialer Richtung nur durch passiv wirkende magnetische Kräfte gehalten.
Der Stator und der Rotorteil sind daher derart auszugestalten, dass eine zum Betrieb der Rotationspumpe genügend grosse, passiv wirkende magnetische Kraft auf den Rotor bewirkt wird. Dies kann durch eine entsprechende Ausgestaltung und geometrische Anordnung der magnetisch wirksamen Komponenten des Rotorteils und des Statorteils erzielt werden, indem zum Beispiel die Zähne des Statorteils in axialer Richtung etwa die gleiche Höhe aufweisen wie der Rotorteil und indem der Durchmesser des Rotorteils zumindest doppelt so gross ausgestaltet ist wie dessen Höhe in axialer Richtung. Die passiv wirkende magnetische Kraft in axialer Richtung kann zudem erhöht werden durch eine permanentmagnetische Vorspannung des Stators sowie des Rotorteils. Die Lagerkraft in axialer Richtung kann zudem durch zusätzliche Lagervorrichtungen wie ein hydrodynamisch wirkendes Lager erhöht werden. Zudem kann es sich als vorteilhaft erweisen eine in axialer Richtung wirkende mechanische Lagervorrichtung vorzusehen, welche bei sehr grossen axialen Kräften als eine Notlauflagervorrichtung dient, um den Rotorteil in einer bestimmten Lage zu halten.

Unter einem lagerlosen Motor wird eine elektrisch ansteuerbare Lager- und Antriebsvorrichtung verstanden, welche einen Rotor sowie einen elektromagnetische Spulen aufweisenden Stator umfasst. Der Rotor des lagerlosen Motors ist nach den an sich bekannten Prinzipien elektrischer Maschinen antreibbar, so nach den Gesetzmässigkeiten eines Synchronmotors, eines Reluktanzmotors oder eines Induktionsmotors. Der Rotor des lagerlosen Motors ist zumindest in einer senkrecht zur Drehachse verlaufenden Ebene durch magnetisch wirkende Kräfte berührungslos im Stator gehalten. Die elektromagnetischen Spulen sind derart ansteuerbar, dass die Lage des Rotors in einer senkrecht zur Drehachse des Rotors verlaufenden Ebene aktiv beeinflussbar ist. Die Lage des Rotors wird mit Sensoren überwacht und die elektromagnetischen Spulen mit einer entsprechend ausgestalteten Ansteuervorrichtung derart regelbar angesteuert, dass der Rotor bezüglich der senkrecht zur Drehachse des Rotors verlaufenden Ebenen berührungslos im Stator gehalten ist. Zudem kann über eine entsprechende Ansteuerung der elektromagnetischen Spulen des Stators ein Drehmoment auf den Rotor erzeugt werden, sodass dieser eine Rotation um dessen axiale Achse erfährt. Ein derartiger sogenannter lagerloser Motor kann somit einen Rotor bezüglich dreier Freiheitsgrade, nämlich der Lage in x- und y-Richtung sowie der Rotation um dessen Achse, aktiv ansteuern. Ein lagerlose Motor aufweisend diese Eigenschaften kann durch unterschiedliche Konstruktionsformen ausgestaltet sein.

Ein lagerloser Motor kann zum Beispiel als ein Reluktanzmotor ausgestaltet sein, indem der Rotor zum Beispiel kreuzförmig ausgestaltet ist, und der Stator aus einer Mehrzahl von in radialer Richtung verlaufenden, in Umfangsrichtung um dem Rotor angeordneten, elektrisch einzeln ansteuerbaren Spulen ausgestaltet ist. Diese Spulen sind derart ansteuerbar, dass der Rotor bezüglich einer senkrecht zur Drehachse verlaufenden Ebene in der Schwebe gehalten wird, und der Rotor zudem um dessen Drehachse rotierend antreibbar ist, indem mit den Spulen ein magnetisches Drehfeld erzeugt wird.

Ein lagerloser Motor kann zum Beispiel ähnlich einem Synchronmotor ausgestaltet sein, indem der Rotor ein in radialer Richtung verlaufender Permanentmagnet aufweist, und der Stator eine Drehfeldwicklung, auch als eine Antriebswicklung bezeichnet, zur Erzeugung eines Drehfeldes aufweist, welches den Rotor um dessen Drehachse rotierend antreibt. Zudem weist der Stator eine Steuerwicklung auf, um die Lage des Rotors in einer senkrecht zur Drehachse verlaufenden Ebene anzusteuern, wobei die Lage der Rotors bzw. der magnetische Fluss mit Sensoren erfasst wird und die Steuerwicklung über eine Ansteuervorrichtung derart angesteuert werden, dass der Rotor in der senkrecht zur Achse des Stators verlaufenden Ebene berührungslos im Stator gehalten wird. In einer Ausführungsform weist ein derart ausgestalteter sogenannter lagerloser Motor eine Antriebswicklung mit einer Polpaarzahl p und eine Steuerwicklung mit einer Polpaarzahl p+1 oder p-1 auf.

Die erfindungsgemässe Rotationspumpe, ausgestaltet als eine Axialpumpe oder eine Zentrifugalpumpe, ist insbesondere geeignet zum Fördern von hochreinen, aggressiven, explosiven oder toxischen Fluide und Flüssigkeiten. Die erfindungsgemässe Rotationspumpe ist ebenfalls geeignet als eine Blutpumpe, betrieben ausserhalb oder innerhalb des menschlichen Körpers.

Ein Vorteil der erfindungsgemässen Rotationspumpe ist darin zu sehen, dass auf Grund der berührungslosen Lagerung der Rotor mit einer sehr hohen Drehzahl betreibbar ist, sodass die Rotationspumpe auch bei sehr kleinen Baugrössen eine hohe Förderleistung aufweist. Ein weiterer Vorteil der erfindungsgemässen Rotationspumpe ist darin zu sehen, dass der Rotor auch mit einer zeitlich variablen Drehzahl betreibbar ist, und das Fluid auch pulsierend förderbar ist. Die Drehzahl des Rotors ist zwischen dem Stillstand und einer sehr hohen Drehzahl frei ansteuerbar, sodass die Rotationspumpe bezüglich der Fördermenge eine grosse Dynamik aufweist und sowohl sehr geringe als auch sehr grosse Fluidmengen förderbar sind, wobei das Fluid insbesondere auch gemäss einer vorgebbaren, pulsierenden Fluidmenge pro Zeiteinheit förderbar ist.

Die Erfindung wird an Hand von mehreren Ausführungsbeispielen im Detail beschrieben. Es zeigen:
- Fig. 1: einen Längsschnitt durch eine Axialpumpe;
- Fig. 1a: einen Schnitt durch einen Stator und ein magnetisch wirksames Rotorteil der Axial- oder Zentrifugalpumpe;
- Fig. 1b,1c,1d: unterschiedliche Lagen des magnetisch wirksamen Rotorteils bezüglich dem Stator einer Axial- oder Zentrifugalpumpe;
- Fig. 2a,2b,2c,2d,2e,2f,2g,2h: unterschiedlich ausgestaltete, magnetisch wirksame Rotorteile;
- Fig. 3a: einen Schnitt durch ein Axialrad entlang der Linie A-A gemäss Fig. 3b;
- Fig. 3b: eine Aufsicht auf das Axialrad gemäss Fig. 3a;
- Fig. 3c: einen Schnitt durch das Axialrad entlang der Linie B-B gemäss Fig. 3a;
- Fig. 4a,4b,4d,4e: Ausführungsformen eines Stators für eine Axial- oder Zentrifugalpumpe;
- Fig. 4f,4g: Ausführungsformen eines Rotors für einen ein Unipolarlager aufweisenden Stator;
- Fig. 4h,4i: ein Wickelschema für eine dreiphasig ausgestaltete Antriebs- bzw. Steuerwicklung;
- Fig. 5a: Ausführungsform einer zusätzlichen, hydrodynamisch wirkenden Lagervorrichtung einer Axialpumpe sowie eine Ansteuervorrichtung;
- Fig. 6a: ein Ausführungsbeispiel eines als Reluktanzläufer ausgestalteten Axialrades;
- Fig. 6b: einen Längsschnitt durch eine einen Reluktanzmotor umfassenden Axialpumpe;
- Fig. 6c: einen Querschnitt durch den Reluktanzmotor gemäss Fig. 6b entlang der Linie C-C;
- Fig. 6d,6e,6f,6g,6h: einen Querschnitt durch verschieden ausgestaltete Axialräder bzw. Reluktanzläufer;
- Fig. 7: eine Aufsicht eines Stators für eine Axial- oder Zentrifugalpumpe;
- Fig. 7a,7b,7c: ein Beispiel einer Ansteuerung der Spulen des Stators gemäss Fig. 7;
- Fig. 8a: einen Längsschnitt durch eine Zentrifugalpumpe entlang der Linie B-B gemäss Fig. 8b;
- Fig. 8b: einen Querschnitt durch die Zentrifugalpumpe gemäss Fig. 8a entlang der Linie A-A;
- Fig. 8i: ein Profil des zwischen dem Gehäuse und dem Rotor auftretenden Druckes des Fluides;

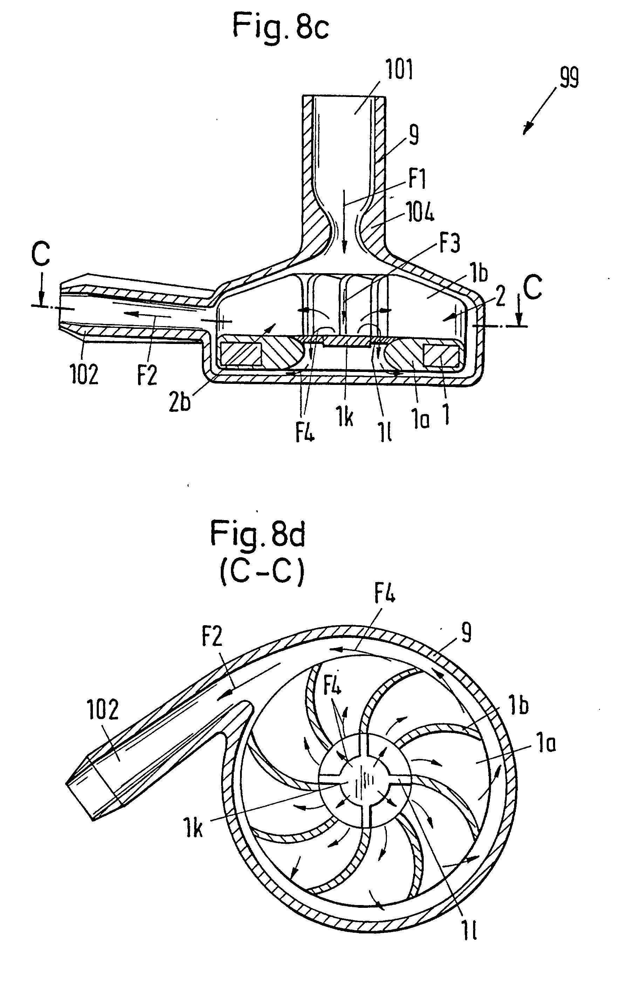

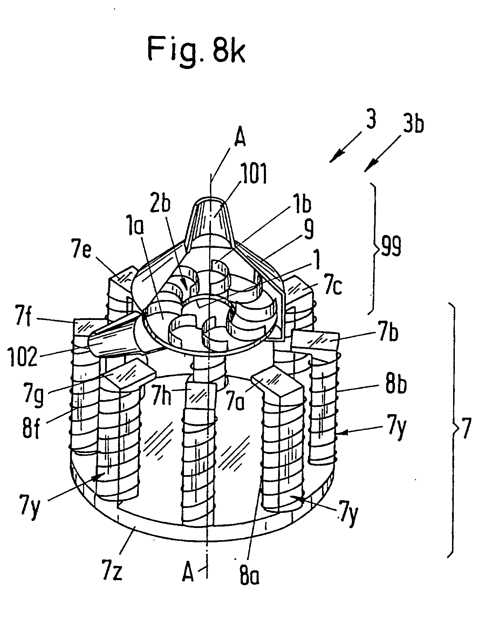

- Fig. 8c-8h: weitere Ausführungsformen eines Zentrifugalpumpenteils;
- Fig. 8k: eine Zentrifugalpumpe mit einem als Tempelmotor ausgebildeten Stator;

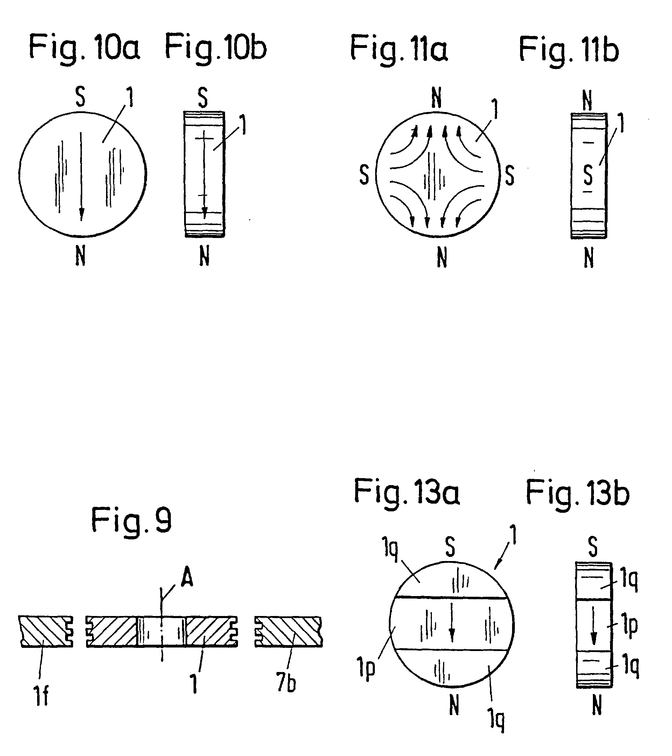

- Fig. 9: einen Querschnitt durch ein magnetisch wirksames Rotorteil;
- Fig. 10a,10b,11a,11b,13a,13b: eine Aufsicht und eine Seitenansicht scheibenförmig ausgestalteter Rotorteile;

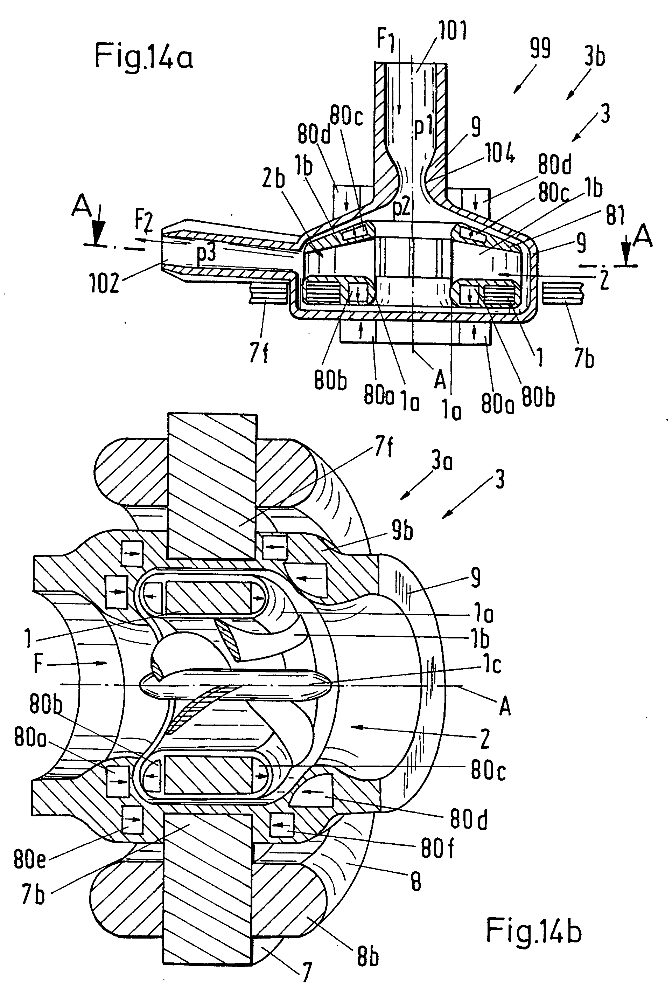

- Fig. 14a: einen Längsschnitt durch eine Zentrifugalpumpe mit einer permanentmagnetisch wirksamen Axialschubkompensation;
- Fig. 14b: ein Längsschnitt durch eine Axialpumpe mit einer permanentmagnetisch wirksamen Axialschubkompensation;

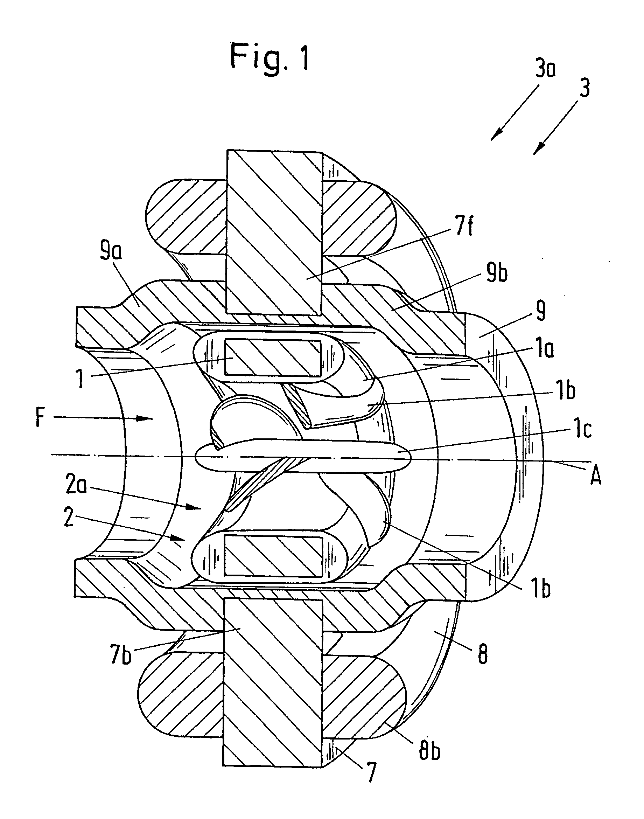

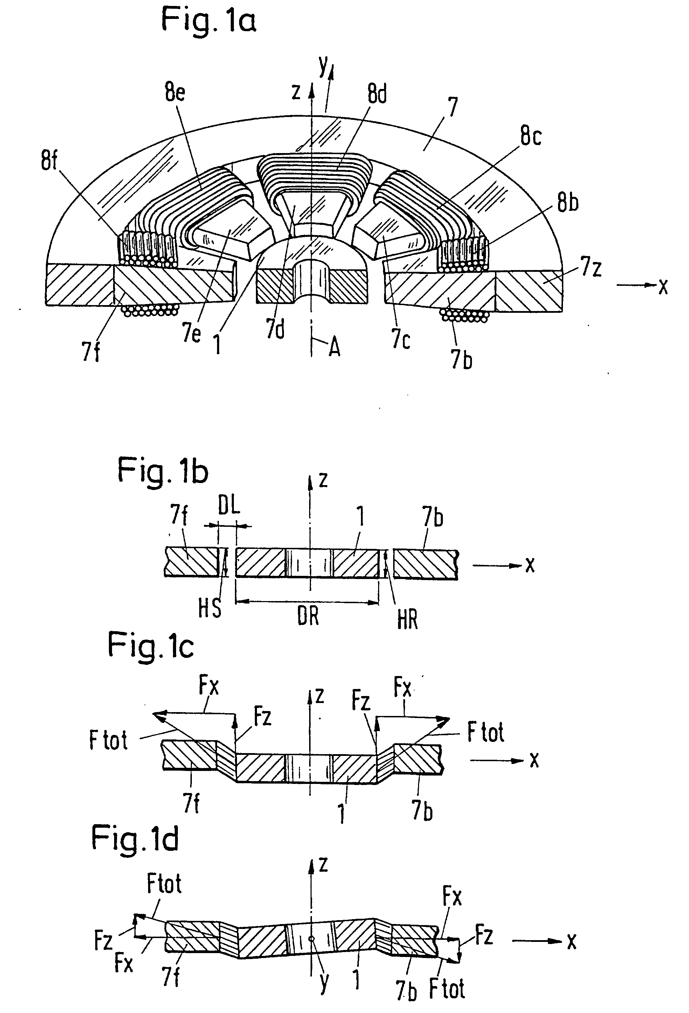

Fig. 1 zeigt einen Längsschnitt durch eine erfindungsgemässe, als Axialpumpe 3a ausgestaltete Rotationspumpe 3, mit einem Gehäuseteil 9, welches Verengungsstellen 9a,9b aufweist. Ausserhalb des Gehäuses 9 ist ein das Gehäuse 9 in Umfangsrichtung umschliessender Stator 7 angeordnet, welcher in radialer Richtung verlaufende Zähne 7b,7f aufweist. Diese Zähne 7b,7f liegen an deren Spitze innerhalb des Mantels des Gehäuseteils 9, derart, dass zwischen der Spitze der Zähne 7b, 7f und dem Innenraum des Gehäuses 9 ein Gehäuseabschnitt liegt, sodass der Innenraum des Gehäuses 9 vollständig getrennt ist vom Stator 7. Der Stator 7 mit Zähnen 7b,7f ist aus einem ferromagnetischen Material gebildet. Die Zähne 7b,7f sind von Spulen 8b umwickelt, wobei die Gesamtheit der Wicklungen 8 dargestellt ist. Innerhalb des Gehäuses 9 ist der Rotor 2, ausgestaltet als ein Axialrad 2a, auch axiales Laufrad genannt, angeordnet. Dieses Axialrad 2a besteht im dargestellten Ausführungsbeispiel aus einem ringförmig ausgestalteten, in radialer Richtung magnetisierten Permanentmagnet 1, welches von einer aus einem Kunststoff bestehenden Rotorummantelung 1a umschlossen ist. Innerhalb der kreisförmigen Ausnehmung des magnetisch wirksamen Rotorteils 1 sind hydrodynamisch verlaufend ausgestaltete Schaufeln 1b sowie ein in bezüglich dem Axialrad 2a in axialer Richtung A verlaufendes Mittelteil 1c angeordnet. Die Schaufeln 1b verlaufen in axialer Richtung A über die Rotorummantelung 1a vorstehend bis zur dem Stator 7 zugewandten Aussenfläche der Rotorummantelung 1a. Die Schaufeln 1b bilden zusammen mit dem Mittelteil 1c ein zur Erzeugung einer axialen Strömung günstiges Flügelrad, wobei das Flügelrad vorteilhafterweise einen möglichst grossen Durchmesser aufweist, um eine grosse Pumpleistung zu bewirken. Vorteilhafterweise sind die Schaufeln 1b nicht nur innerhalb des Innendurchmessers des Rotorteils 1 verlaufend angeordnet, sondern verlaufen, wie in Fig. 1 dargestellt, in axialer Richtung A über die Rotorummantelung 1a vorstehend bis annähernd zur Wand des Gehäuses 9. Dadurch wird beinahe der gesamte Durchmesser des Innenraumes des Gehäuses 9 genutzt zur Anordnung des Flügelrades beziehungsweise zur Förderung des Fluides. Die Rotorummantelung 1a sowie die Schaufeln 1b und das Mittelteil 1c als auch das Gehäuse 9 sind aus einem nicht ferromagnetischen Material wie einem Kunststoff, Metall wie Titan, Keramik oder einem biokompatiblen Werkstoff wie Polycarbonat gefertigt. Das Axialrad 2a wird durch den magnetisch wirksamen Rotorteil 1 über magnetisch wirkende Kräfte vom Stator 7 berührungslos innerhalb des Gehäuses 9 gehalten, wobei zugleich ein auf den Rotorteil 1 wirksames Drehmoment erzeugbar ist, um das Axialrad 2a um die Rotationsachse A drehend anzutreiben. Nicht dargestellt sind Sensoren 15 zur Erfassung der Lage des Axialrades 2a beziehungsweise des Rotorteils 1. Diese Sensoren 15 sind vorzugsweise entweder ausserhalb des Gehäuses 9 oder innerhalb des Mantels des Gehäuses 9 angeordnet, um die Lage des Rotorteils 1 oder den magnetischen Fluss berührungslos zu messen. Als Messprinzip der Sensoren 15 ist zum Beispiel ein Wirbelstromsensor, ein induktiver Sensor oder ein Hallelement mit einem Permanentmagnet geeignet. Die Lage des Axialrades 2a ist in einer etwa senkrecht zur Rotationsachse A verlaufenden Ebene berührungslos ansteuerbar durch eine entsprechende Ansteuerung der im Stator 7 angeordneten Wicklungen 8a,8b,8c,8d,8e,8f. Die Wicklungen umfassen eine Antriebswicklung WA mit einer Polpaarzahl p und eine Steuerwicklung WS mit einer Polpaarzahl p+1 oder p-1.

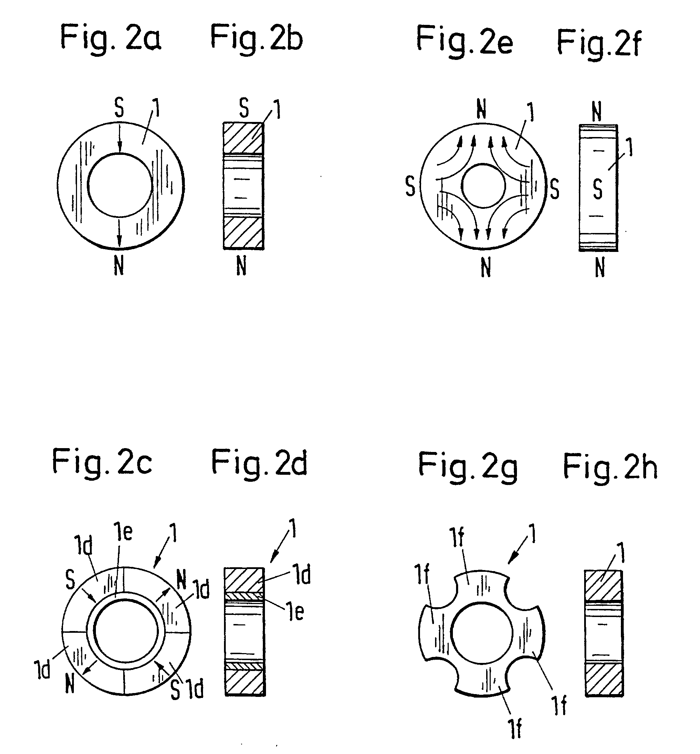

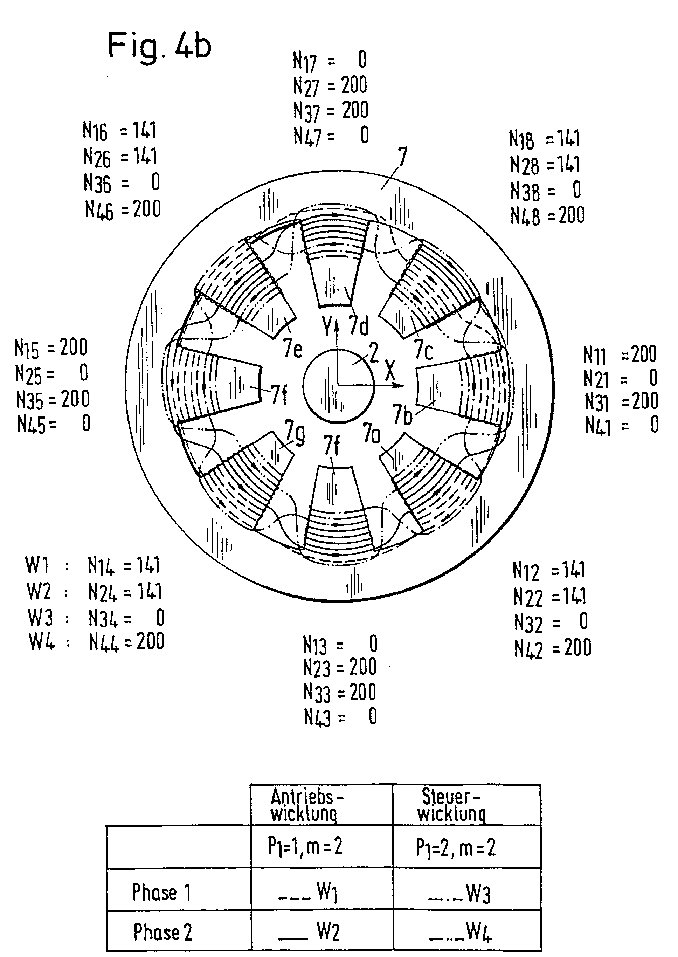

Fig. 1a zeigt eine perspektivische Ansicht eines Schnittes durch einen Stator 7 sowie ein magnetisch wirksames Rotorteil 1 der Rotationspumpe 3, ausgestaltet als Axialpumpe 3a oder als Zentrifugalpumpe 3b. Die magnetisch nicht wirksamen Teile der Rotationspumpe 3, wie das Gehäuse 9 oder die Rotorummantelung 1a und die Schaufeln 1b sind zum besseren Verständnis der Anordnung nicht dargestellt. Der Stator 7 weist ein ringförmiges, als Rückschlusseisen 7z ausgestaltetes Teil auf, an welchem die Zähne 7b,7c,7d,7e,7f in radialer Richtung verlaufend angeordnet sind. Das Rückschlusseisen 7z sowie die Zähne 7b,7c,7d,7e,7f bestehen aus einem ferromagnetischen Metall. Der magnetisch wirksame Rotorteil 1 besteht, wie in Fig. 2a und Fig. 2b dargestellt, aus einem ringförmigen, in radialer Richtung polarisierten Permanentmagnet. Die Spulen 8b,8c,8d,8e,8f weisen zwei voneinander unabhängig ansteuerbar ausgestaltete Teilwicklungen auf, wobei die eine Teilwicklung als eine Antriebswicklung WA mit einer Polpaarzahl p und die andere Teilwicklung als eine Steuerwicklung WS mit einer Polpaarzahl von p+1 oder p-1 ausgestaltet ist. Eine derartige Anordnung der Wicklungen ist in Fig. 4b im Detail beschrieben. Die Antriebswicklung WA und der magnetisch wirksame Teil des Rotors 1 wirken vergleichbar einer Synchronmaschine zusammen, indem mit der zweiphasig ausgestalteten Antriebswicklung WA im Stator 7 ein magnetisches Drehfeld erzeugt wird, welchem der Rotor 1 folgt, sodass der Rotor 1 um dessen Achse A drehend angetrieben wird. Die Lage des Rotors 1 wird mit nicht dargestellten Sensoren 15 erfasst und die dreiphasig ausgestalteten Steuerwicklungen WS, unter Berücksichtigung des durch die Antriebswicklung WA erzeugten magnetischen Feldes, derart angesteuert, dass der Rotor 1 in einer zur Achse A senkrechten Ebene, daher in der x- und y-Richtung, berührungslos im Stator 7 gehalten wird. Der Rotor 1 ist somit bezüglich dreier Freiheitsgrade, nämlich der Lage in x- und y-Richtung sowie der Rotation um die Achse A, aktiv ansteuerbar.

Fig. 1b zeigt in einer Seitenansicht der Fig. 1a den Rotor 1 in einer Normallage zwischen den Statorzähnen 7b und 7f angeordnet, wobei der Rotorteil 1 einen Durchmesser DR und eine Höhe HR aufweist, der Statorzahn 7f oder 7b an der dem Rotorteil 1 zugewandten Seite eine Höhe HS aufweist, und der Spalt zwischen dem Statorzahn 7f oder 7b und dem Rotor 1 eine Distanz DL aufweist. Fig. 1c zeigt den Rotor 1 in Richtung z aus der Normallage ausgelenkt. Der permanentmagnetische Rotor 1 bewirkt ein magnetisches Feld, welches ausgehend vom Rotor 1 über den Luftspalt zum Statorzahn 7b verläuft, und über das Rückschlusseisen 7z, den Statorzahn 7f und den Luftspalt wieder in den Rotor 1 mündet, wobei ein Teil des gesamten magnetischen Flusses auch über die Statorzähne 7c,7d,7e verläuft. Dieser permanentmagnetisch vorgespannte Flusskreis bewirkt bei einer Auslenkung des Rotors 1 in z-Richtung aus der Normallage eine passiv wirkende, rückstellende Reluktanzkraft Ftot, die sich aus einer in z-Richtung verlaufenden Komponente Fz und einer in x-Richtung verlaufenden Komponente Fx zusammensetzt. Die Kraftkomponente Fz wirkt stabilisierend auf die Lage des Rotors 1, denn diese wirkt bei einer Auslenkung des Rotors 1 in z-Richtung immer in zur Auslenkung entgegengesetzten Richtung und ist bestrebt den Rotor 1 in die Normallage zu bringen.

Fig. 1d zeigt den Rotor 1 in einer bezüglich der y-Achse verkippten Lage. In dieser Lage übt eine passiv wirkende, rückstellende Reluktanzkraft Ftot ein der Verkippung entgegengesetztes Drehmoment auf den Rotor 1 aus, sodass die rückstellende Kraftkomponente Fz stabilisierend auf die Lage des Rotors 1 wirkt und bestrebt ist, den Rotor 1 in die Normallage zu bringen. Derselbe rückstellende Effekt gilt bei einer Verkippung des Rotors 1 um die x-Achse. Somit ist die Lage des Rotors 1 bezüglich dreier Freiheitsgrade, nämlich einer translatorischen Bewegung in z-Richtung sowie einer Verkippung um die x- und y-Achse durch passiv wirkende Reluktanzkräfte stabilisiert. Der Rotor 1 ist somit durch magnetisch wirkende Kräfte einerseits berührungslos im Stator 7 gelagert und andererseits um dessen Achse A motorisch antreibbar, wobei drei Freiheitsgrade der Lage des Rotors 1 ansteuerbar sind, und die drei weiteren Freiheitsgrade der Lage des Rotors 1 durch passiv wirkende Reluktanzkräfte in einer stabilen Lage gehalten werden. Damit diese passiv wirkenden Reluktanzkräfte auftreten ist der Rotorteil 1 und die Form der Zähne 7b,7c,7d,7e,7f des Stators 7 entsprechend geometrisch gegenseitig angepasst auszugestalten und anzuordnen. Die Zähne 7b,7c,7d,7e,7f weisen auf der dem Rotor 1 zugewandten Seite vorzugsweise eine Höhe HS auf, die gleich der Höhe HR oder ungefähr gleich der Höhe HR des Rotorteils 1 ist. Der Durchmesser DR des Rotorteils 1 ist derart ausgestaltet, dass dieser mehr als doppelt so gross ist wie die Höhe HR des Rotorteils 1. Durch diese gegenseitig angepasste geometrische Ausgestaltung und Anordnung von Stator 7 und Rotor 1 werden die rückstellenden, passiv wirkenden Reluktanzkräfte ermöglicht. Diese passiv wirkenden Reluktanzkräfte können erhöht werden durch einen geringen Abstand DL, wobei dieser Abstand, wie aus Fig. 1 ersichtlich, durch die Dicke der Gehäusewand 9 und der Rotorummantelung 1a bestimmt wird. Die passiv wirkende Reluktanzkräfte lassen sich ebenfalls durch eine permanentmagnetische Vorspannung des magnetischen Kreises erhöhen, indem zum Beispiel im Stator 7 eine zusätzliche Spule oder ein zusätzliches Permanentmagnet angeordnet ist zur Erhöhung des magnetischen Flusses durch den Rotorteil 1.

Mit der erfindungsgemässe Axialpumpe 3a wird ein in axialer Richtung beziehungsweise in z-Richtung wirkender Schub auf das in Flussrichtung F geförderte Fluid bewirkt. Diese am Axialrad 2a angreifende Schubkraft in z-Richtung muss durch eine in entgegengesetzter Richtung wirkende, passive Reluktanzkraft kompensiert werden. Daher ist für den Betrieb der Axialpumpe 3a die maximal in z-Richtung wirkende, passive Reluktanzkraft Fz von entscheidender Bedeutung, da bei einem Überschreiten dieser Maximalkraft der Rotorteil 1 aus der Gleichgewichtslage im Stator 7 gerissen würde. Um ein derartiges Herausreissen zu vermeiden weist das Gehäuse 9 gemäss dem Ausführungsbeispiel Fig. 1 Verengungsstellen 9a, 9b auf, welche die Bewegungsfreiheit des Axialrades 2a in axialer Richtung A begrenzen. Bei einer starken Auslenkung des Axialrades in z-Richtung würde das Axialrad 2a im Bereich der Verengungsstellen 9a, 9b mit dem Gehäuse 9 in Berührung kommen, womit die maximale Auslenkung begrenzt ist. Der magnetisch wirksame Rotorteil 1 bleibt im magnetisch wirksamen Einflussbereich des Stators 7, sodass das Axialrad 2a über magentisch wirkende Kräfte wieder in die Normallage rückführbar ist.

Fig. 2e zeigt in einer Aufsicht ein weiteres, ringförmig ausgestaltetes Rotorteil 1 mit zwei Polpaaren bzw. zwei Südpolen S und zwei Nordpolen N. Fig. 1f zeigt eine Seitenansicht des Rotorteils gemäss Fig. 2e mit zwei Polpaaren und vier Polen S,N. Fig. 2c zeigt ein weiteres Ausführungsbeispiel eines Rotorteils 1, das vier Schalenmagnete 1d umfasst, welche anschliessend an ein ringförmiges Rückschlusseisen 1e angeordnet sind, wobei die Aussenflächen des Rotorteils 1 bzw. der Schalenmagnete 1d abwechslungsweise einen Südpol S und einen Nordpol N ausbilden. Fig. 2d zeigt einen Schnitt durch die Mitte des Rotorteils 1 gemäss Fig. 2c mit Schalenmagnet 1d und Rückschlusseisen 1e.

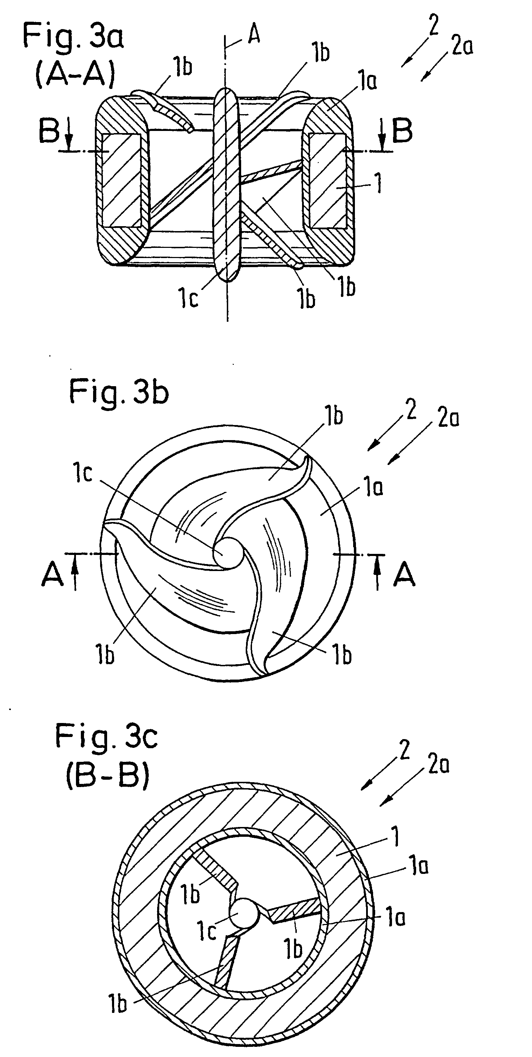

Fig. 3a zeigt einen Längsschnitt durch das Axialrad 2a, wie dies im Ausführungsbeispiel gemäss Fig. 1 Verwendung findet. Der magnetisch wirksame Rotorteil 1 ist als ein Permanentmagnet, wie in Fig. 2a und Fig. 2b dargestellt, ausgeführt. Der Rotorteil 1 ist von einer Ummantelung 1a eingeschlossen und weist zudem mit dem Rotorteil 1 fest verbundene Schaufeln 1b mit Mittelteil 1c auf. Die Schaufeln 1b sind derart hydrodynamisch verlaufend ausgestaltet, dass ein in axialer Richtung A wirkender Schub auf ein Fluid erzeugbar ist. Fig. 3b zeigt eine Aufsicht auf das Axialrad 2a gemäss Fig. 3a, wobei die Rotorummantelung 1a, der Verlauf der Schaufeln 1b sowie das Mittelteil 1c ersichtlich ist. Fig. 3c zeigt einen Schnitt entlang der Linie B-B gemäss Fig. 3a. Aus Fig. 3c ist der ringförmige Verlauf des magnetisch wirksamen Rotorteils 1 ersichtlich, sowie die insbesondere innerhalb des Ringes verlaufenden Schaufeln 1b sowie das Mittelteil 1c.

Fig. 4b zeigt die Wicklung 8 des Stators 7 gemäss dem Ausführungsbeispiel Fig. 1 im Detail. An den Zähnen 7a,7b,7c,7d,7e,7f,7g,7h des Stators 7 sind eine zweiphasige Antriebswicklung WA (Wicklungen W1 und W2) mit einer Polpaarzahl 1 zur Erzeugung eines magnetischen Drehfeldes sowie eine zweiphasige Steuerwicklung WS mit einer Polpaarzahl 2 (Wicklungen W3,W4) zum Ansteuern der Lage des Rotors 2 in x- und y-Richtung angeordnet. Die Antriebswicklung WA weist eine Polpaarzahl p=1 und die Steuerwicklung WS eine Polpaarzahl p=2 auf. Nxx bedeutet die Windungszahlen der einzelnen Wicklungen W1,W2,W3,W4 für ein Ausführungsbeispiel, wobei N11,N12,N13, auch als N1x bezeichnet, die Anzahl Windungen am betreffenden Zahn 7a,7b,7c,7d,7e,7f,7g,7h der Wicklung W1 bezeichnet. Weiter sind mit N2x, N3x, N4x die entsprechende Anzahl Windungen der Wicklungen W2,W3,W4 an den entsprechenden Zähnen dargestellt.

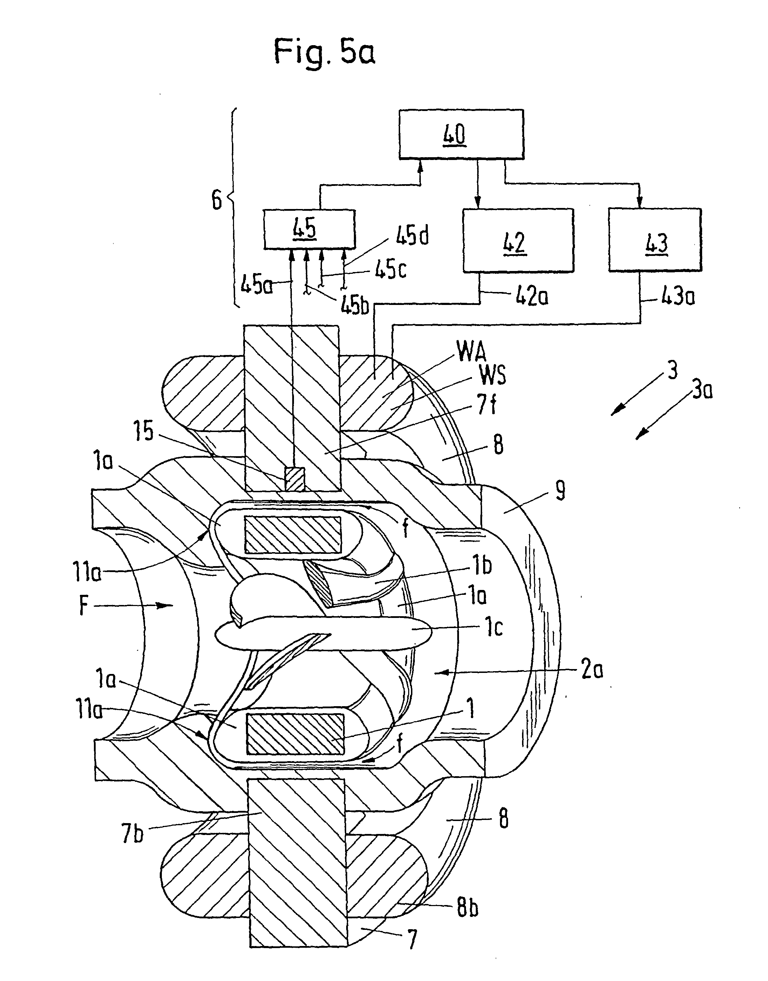

Fig. 5a zeigt im oberen Teil eine Ansteuervorrichtung 6 zur Ansteuerung der Rotationspumpe 3, ausgeführt als eine Axialpumpe 3a gemäss dem Ausführungsbeispiel Fig. 1 oder Fig. 5a. Die Lage des Axialrades 2a wird mit Hilfe eines an der Oberfläche des Zahnes 7f angeordneten Sensor 15 bestimmt, wobei das Sensorsignal über eine Signalleitung 45a einer Signalauswertevorrichtung 45 zugeführt ist, welche Auswertevorrichtung 45 die Signale weiterer, nicht dargestellter Sensoren zur Überwachung des Axialrades 2, die über Signalleitungen 45b,45c,45d zugeführt sind, auswertet, um die Lage sowie die Drehzahl des Axialrades 2 im Gehäuse 9 zu bestimmen. Die Werte werden einer Regelvorrichtung 40 zugeführt, welche einen Mikrocomputer umfasst, wobei die Regelvorrichtung 40 für die Antriebsund Steuerwicklung einen Sollwert berechnet, und diese Sollwerte dem Stromsteller 42 der Steuerwicklung WS sowie dem Stromsteller 43 der Antriebswicklung WA vorgibt. Die Steuerwicklung WS ist über eine elektrische Leitung 42a mit dem Stromsteller 42 und die Antriebswicklung WA über eine elektrische Leitung 43a mit dem Stromsteller 43 verbunden.

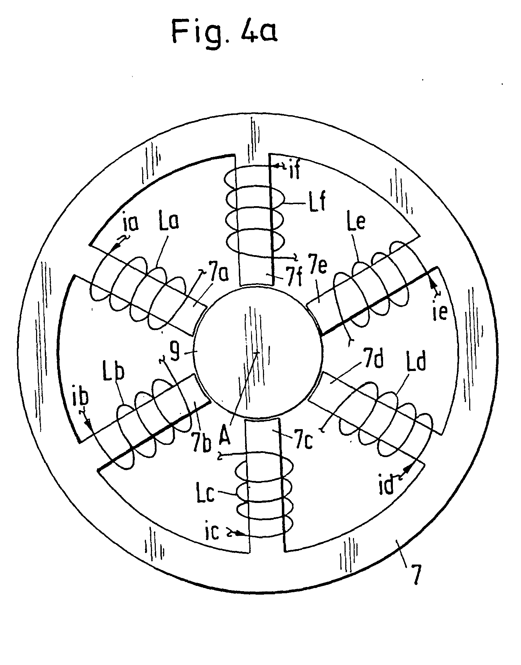

Fig. 4a zeigt eine weitere Ausführungsform eines Stators 7 mit sechs in radialer Richtung verlaufenden Zähnen 7a,7b,7c,7d,7e, welche einzeln ansteuerbare Spulen La, Lb, Lc, Ld, Le, Lf aufweisen. Ein nicht dargestelltes, gemäss Fig. 2a ausgestaltetes, magnetisch wirksames Rotorteil 1 ist innerhalb des Gehäuses 9 angeordnet. Jede der Spulen La, Lb, Lc, Ld, Le, Lf ist mit einer Stellvorrichtung verbunden, wobei die Spulen von einer übergeordneten Ansteuervorrichtung 40 derart angesteuert werden, dass der Rotor 1 berührungslos bezüglich einer senkrecht zur Drehachse A verlaufenden Ebene gehalten wird und zudem auf den Rotor 1 ein antreibendes Drehmoment bewirkt wird.

Der Stator 7 gemäss Fig. 4a wie auch der Stator 7 gemäss Fig. 1a ist auch in der Funktion eines Reluktanzmotors betreibbar, indem ein zum Beispiel gemäss Fig. 2g ausgestalteter Rotor 1 verwendet wird. Dieses, kreuzförmig vorstehend ausgestaltete Teilabschnitte 1f aufweisende, magnetisch wirksame Rotorteil 1 ist ferromagnetisch ausgestaltet, weist jedoch keine Permanentmagnetisierung auf. Die Rotation des Rotors 1 um die Drehachse A wird durch Reluktanzkräfte erzeugt indem die einzelnen Spulen La,Lb,Lc,Ld,Le,Lf entsprechend zeitlich nacheinander angesteuert werden. Der Rotor 1 wird zudem durch eine entsprechende Ansteuerung der Spulen La,Lb,Lc,Ld,Le,Lf berührungslos in der Schwebe gehalten. Fig. 2h zeigt einen Längsschnitt durch den kreuzförmigen Rotor 1 gemäss Fig. 2g.

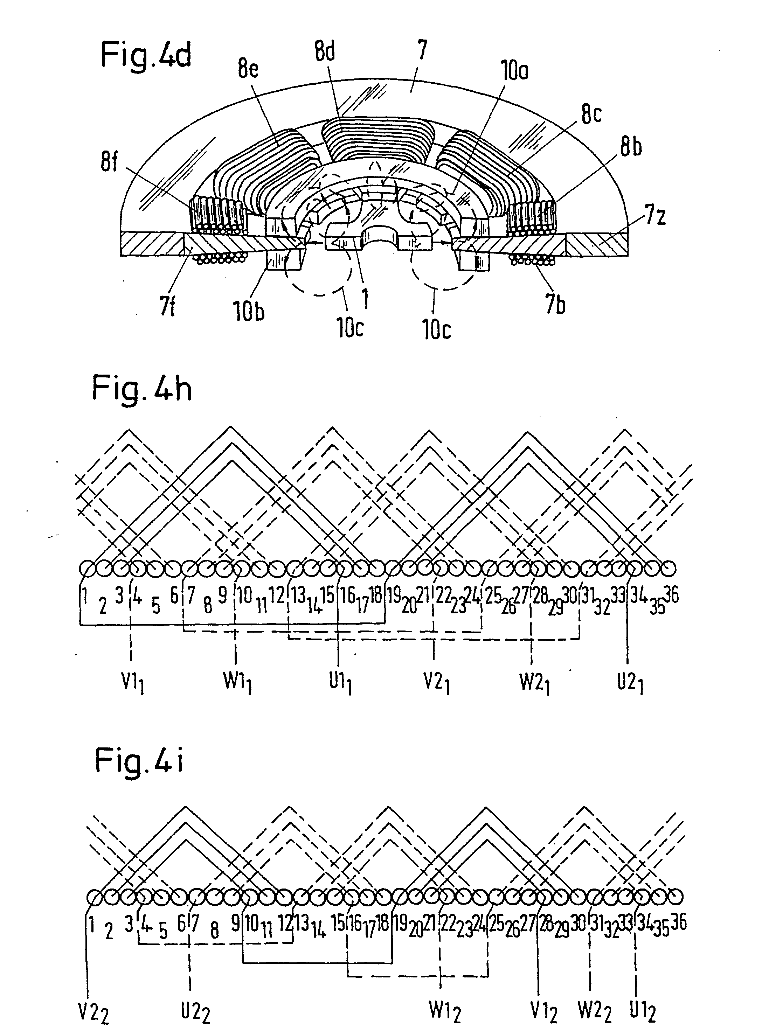

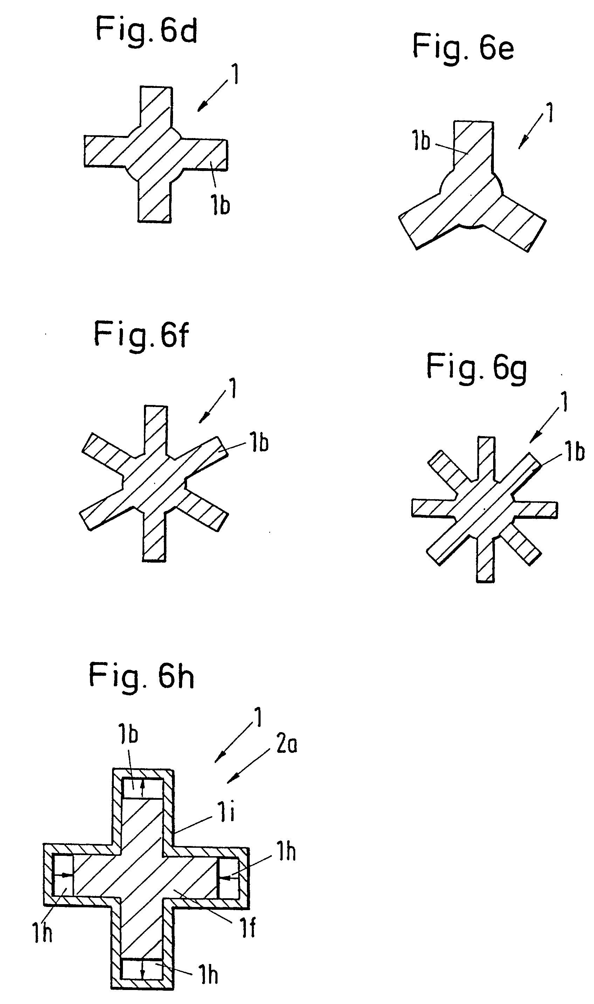

Fig. 4d zeigt ein weiteres Ausführungsbeispiel eines durch einen Stator 7 berührungsfrei gehaltenen und angetriebenen, magnetisch wirksamen Rotorteils 1. Das Rotorteil 1 ist als ein Synchronreluktanzläufer in einer Ausführung gemäss Fig. 2g oder Fig. 6f ausgestaltet. Im Endbereich der Zähne 7a,7b,7c,7d,7e,7f ist beidseitig der Zähne je ein in axialer Richtung A polarisierter, ringförmig ausgestalteter Permanentmagnet 10a, 10b angeordnet. Diese Permanentmagnete 10a, 10b erzeugen einen durch die Feldlinien 10c angedeuteten Unipolarfluss, wobei der zwischen dem Rotor 1 und den Zähnen 7a, 7b, 7c, 7d, 7e, 7f verlaufende magnetische Unipolarfluss in radialer Richtung nach aussen verläuft. Durch diesen Unipolarfluss wird die passiv wirkende Reluktanzkraft beziehungsweise die in den Figuren 1c und 1d dargestellte Rückstellkraft Ftot erhöht, sodass der Rotor 1 stabiler im Stator 7 gelagert ist oder eine grössere, in axialer Richtung A wirkende Schubkraft durch das Axialrad 2a erzeugbar ist. Dabei kann die Steuerwicklung WS 2-polig und die Antriebswicklung WA 4-oder 6-polig ausgeführt sein. Zudem sind bei gleichbleibender Durchflutung der Wicklungen auch ein erhöhtes Drehmoment und erhöhte Radiallagerkärfte erzeugbar.

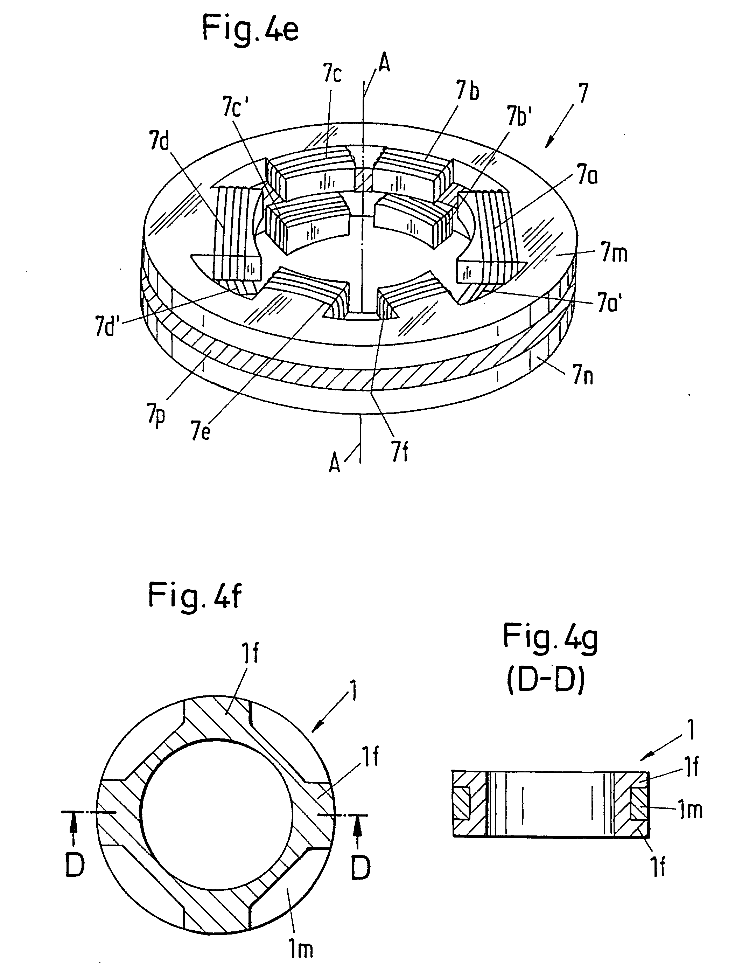

Fig. 4e zeigt ein weiteres Ausführungsbeispiel eines Stators 7, welcher einen zusätzlichen Unipolarfluss zur stabileren Lagerung des Rotors 2 aufweist. Der Stator 7 besteht aus einem ersten Statorteil 7m mit Zähnen 7a,7b,7c,7d,7e,7f, welche jeweils eine Wicklung aufweisen, sowie aus einem zweiten Statorteil 7n mit Zähnen 7a',7b',7c',7d',7e',7f', welche jeweils eine Wicklung aufweisen, sowie ein zwischen den beiden Statorteilen 7m,7n angeordnetes, ringförmig ausgestaltetes, in axialer Richtung A polarisiertes Permanentmagnet 7p. Fig. 4f zeigt in einer Aufsicht und Fig. 4g in einer Schnittdarstellung entlang der Linie D-D ein Ausführungsbeispiel eines Rotorteils 1 für einen Stator 7 gemäss dem Ausführungsbeispiel 4e. Der Rotorteil 1 umfasst ein zylinderförmiges Eisenteil mit kreuzförmig vorstehenden Teilen 1f, sodass sich, wie aus Fig. 4g ersichtlich, ein gegen die Zähne des Stators 7 hin u-förmig ausgestalteter, ferromagnetisches Rotorteil 1 ergibt. Der in dieser ausgebildeten Nut eingelassene Sensorring 1m besteht aus einem nicht ferromagnetischen Metall. Bei im Stator 7 gemäss Fig. 4e eingesetztem Rotorteil 1 wird ein magnetischer Unipolarfluss aufgebaut, der ausgehend vom Permanentmagent 7p über die Zähne 7a,7b,7c,7d,7e,7f,7g zum Rotorteil 1 und von diesem über die Zähne 7a',7b',7c',7d',7e',7f',7g' wieder zum Permanentmagent 7p verläuft. Dieser Unipolarfluss erzeugt eine stabilisierende Wirkung bezüglich einer Auslenkung des Rotorteils 1 in axialer Richtung A. Zusammen mit einer Wicklung der Polpaarzahl 1 sowie einer Regelvorrichtung ist die radiale Lage des Rotors stabilisierbar. Sind die Wicklungen der Polpaarzahl 1 im ersten Statorteil 7m und im zweiten Statorteil 7n getrennt ansteuerbar, so kann durch eine entsprechende Ansteuerung dieser Wicklungen auch die Verkippung des Rotors 1 aktiv, das heisst regelbar stabilisiert werden. Mit einer Wicklung mit derselben Polpaarzahl wie der Rotor ist ein Drehmoment erzeugbar. Die Sensoren 15 zur Erfassung der Lage des Rotors 1 können in axialer Richtung zwischen den beiden Statorteilen 7n,7m liegend angeordnet sein.

Fig. 5a zeigt eine Anordnung zur Erhöhung der Schubkraft des Axialrades 2a. Das Gehäuse 9 weist auf der linken Seite einen der Geometrie der Rotorummantelung 1a angepasst ausgestalteten Teilbereich 11a auf, welcher zusammen mit der Rotorummantelung 1a ein hydrodynamisches, in axialer Richtung A wirkendes Axiallager bildet. Das an der rechten Seite der Axialpumpe 3 unter höherem Druck stehende Fluid, insbesondere eine Flüssigkeit, strömt in einem Teilstrom f zwischen dem Axialrad 2a und dem Gehäuse 9 in Strömungsrichtung f fliessend zur linken Seite der Axialpumpe 3, der Saugseite, zurück, wobei sich zumindest im Teilbereich 11a ein hydrodynamisches Axiallager ausbildet. Zudem kann der Spalt zwischen dem Axialrad 2a und dem Gehäuse 9 im Bereich des Stators 7 derart breit ausgestaltet sein, dass sich auch in diesem Bereich ein Lagerspalt für ein hydrodynamisches Radiallager des Axialrades 2 ergibt. Die in Fig. 5a dargestellte hydrodynamische oder fluiddynamische Lagerung übt eine zusätzliche, stabilisierende Wirkung auf das Axialrad 2a aus, sodass das Axialrad 2a auch bei relativ grossen, am Axialrad 2a angreifenden Kräften sicher und bezüglich dem Gehäuse 9 berührungslos gelagert ist.

Eine zusätzlich zur magnetischen Lagerung hydro- bzw. fluiddynamisch wirkenden Lagerung des Axialrades 2 innerhalb des Gehäuse 9 ist durch eine Vielzahl von Ausgestaltungsformen erzielbar. Beispielsweise kann die Rotorummantelung 2 auf der dem Stator 7 zugewandten Aussenfläche eine wendelförmig verlaufende Rille aufweisen, so dass ein hydrodynamisch wirkendes Spurlager ausgebildet ist. Durch diese Massnahme wird zusätzlich der Fluss des von der Druckseite zur Saugseite hin fliessenden Fluides f entweder verstärkt, gemindert, unterbunden oder umgelenkt, da das sich im Spalt befindliche Fluid entsprechend der Steigung der wendelförmigen Rille sowie der Drehrichtung und der Drehzahl des Rotors gefördert wird. Mit ähnlichen, an der Stirnseite des Rotors angeordneten Rillen kann zudem die Wirkung des hydrodynamischen, in axialer Richtung wirkenden Lagers verbessert werden.

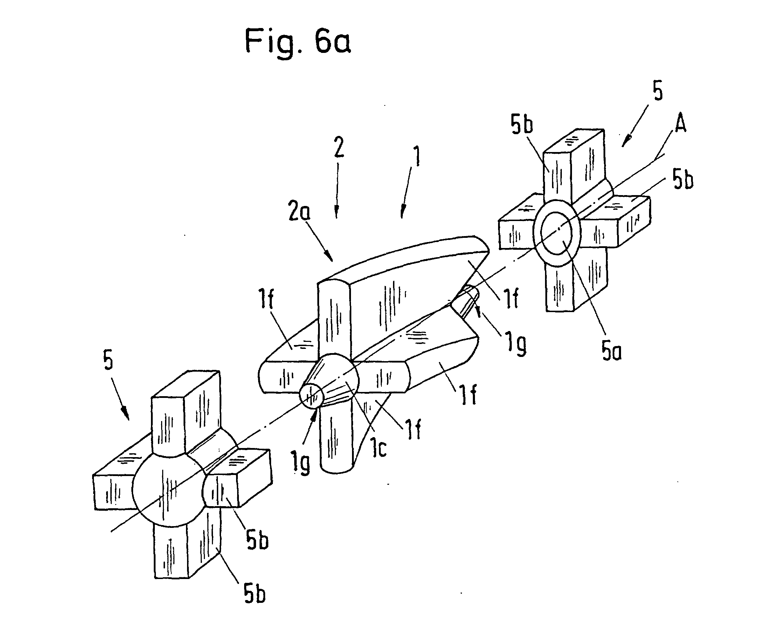

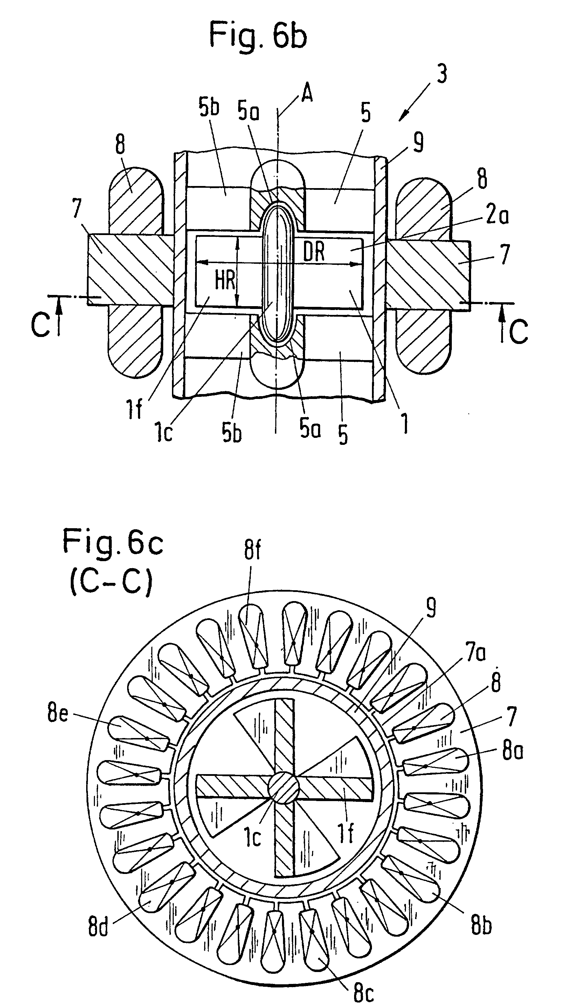

In Fig. 6a ist ein Axialrad 2a für einen nach dem Prinzip eines Reluktanzmotors wirkenden Stator 7 dargestellt. Der magnetisch wirksame Rotorteil 1 bildet zugleich das Axialrad 2a, indem der Rotorteil 1 aus einem ferromagnetischen Werkstoff ausgestaltet ist, jedoch nicht permanentmagnetisiert ist, und indem die kreuzförmig vorstehenden Abschnitte 1f des Rotorteils 1 in axialer Richtung A eine Verwindung aufweisen, welche derart ausgestaltet ist, dass die Abschnitte 1f zugleich die Schaufeln 1b des Axialrades 2a bilden. Der Verlauf der Abschnitte 1f ist entsprechend hydrodynamisch optimiert ausgestaltet, sodass durch diese Abschnitte 1f ein in axialer Richtung auf das Fluid wirkender Schub erzeugbar ist. Der Durchmesser DR des Axialrades 2a ist mindestens doppelt so gross wie die axiale Höhe HR des Axialrades 2a. Das Axialrad 2a ist durch magnetisch wirkende Kräfte berührungslos im Stator 7 antreibbar und gehalten. Neben dem Axialrad 2a ist beidseitig in axialer Richtung A eine Notlagervorrichtung 5 beabstandet angeordnet. Bei einem Ausfall der magnetischen Lagerung oder bei sehr grossen Schubkräften in axialer Richtung wird das Axialrad 2a durch in axialer Richtung vorstehende Notlauflagerteile 1g in einer Notlauflagerstelle 5a gehalten. Die Notlauflagervorrichtung 5 weist kreuzförmig angeordnete Stege 5b beziehungsweise Leitschaufeln 2b auf, welche mit der Gehäusewand 9 in Wirkverbindung sind und von dieser gehalten sind. Die Stege 5b bilden einen Pumpenstator beziehungsweise Leitschaufeln. Fig. 6b zeigt einen Längsschnitt durch eine Axialpumpe 3 mit einem in Fig. 6a dargestellten Axialrad 2a. Die Axialpumpe 3 weist ein Gehäuse 9 auf, das von einem Stator 7 mit Spulen 8 umgeben ist. Im inneren des Gehäuses 9 ist der magnetisch wirksame Rotorteil 1 mit Schaufeln 1b und Mittelteil 1c berührungslos gelagert und antreibbar, wobei zwei Notlauflagervorrichtungen 5 in axialer Richtung A versetzt neben dem Rotorteil 1 beziehungsweise neben dem Axialrad 2a angeordnet sind. Der magnetisch wirksame Rotorteil 1 weist einen Durchmesser DR auf, welcher mehr als doppelt so gross ist wie die Höhe HR des Rotorteils 1. Fig. 6c zeigt einen Querschnitt durch die Axialpumpe 3 gemäss Fig. 6b entlang der Linie C-C. Im Stator 7 sind in axialer Richtung A verlaufende Statornuten mit Wicklungen 8 angeordnet, welche Wicklungen 8 eine Mehrzahl separat ansteuerbaren Wicklungen 8a, 8b, 8c, 8d, 8e, 8f, umfasst, die derart ansteuerbar sind, dass ein magnetisches Drehfeld erzeugbar ist. Die Wicklungen 8 können auch, wie in Fig. 4h, 4i dargestellt, als eine dreiphasige Antriebswicklung WA mit einer Polpaarzahl p und eine dreiphasige Steuerwicklung WS mit einer Polpaarzahl p+1 oder p-1 ausgestaltet sein. Zwischen dem Stator 7 und dem Axialrad 2a ist ein nicht ferromagnetisches Gehäuse 9 angeordnet. Im dargestellten Ausführungsbeispiel ist der magnetisch wirksame Rotorteil 1 mit einem Mittelteil 1c und sternförmig in radialer Richtung abstehenden Teilen 1f ausgestaltet. Die Figuren 6d,6e,6f,6g zeigen weitere, derartige Schnitte des magnetisch wirksamen Rotorteils 1 in unterschiedlichen Ausgestaltungen. In diesen Figuren 6d,6e,6f,6g ist der axiale Verlauf der Schaufeln 1b nicht dargestellt. Die Schaufeln 1b gemäss Fig. 6d,6e,6f,6g weisen in axialer Richtung A einen hydrodynamisch wirksamen Verlauf aus, indem die Schaufeln 1b eine entsprechende Verwindung aufweisen.

Fig. 6h zeigt einen Querschnitt durch ein weiteres Ausführungsbeispiel eines magnetisch wirksamen Rotorteils 1, das ebenfalls als ein Axialrad 2a ausgestaltet ist. Das Rotorteil 1 umfasst einen kreuzförmig ausgestalteten Körper 1f bestehend aus einem ferromagnetischen jedoch nicht permanentmagnetisierten Metall, an dessen Spitzen jeweils ein Permanentmagnet 1h angeordnet ist. Der Körper 1f sowie die Permanentmagnete 1h sind von einer gemeinsamen Ummantelung 1i umschlossen, wobei die Ummantelung 1i aus einem nicht ferromagnetischen Material wie einem Metall z.B. Titan oder einem Kunststoff, insbesondere einem biokompatiblen Kunststoff wie Polycarbonat, besteht. Dieses Axialrad 2a ist zum Beispiel mit einem Stator 7 gemäss dem Ausführungsbeispiel Fig. 6c oder Fig. 4a betreibbar.

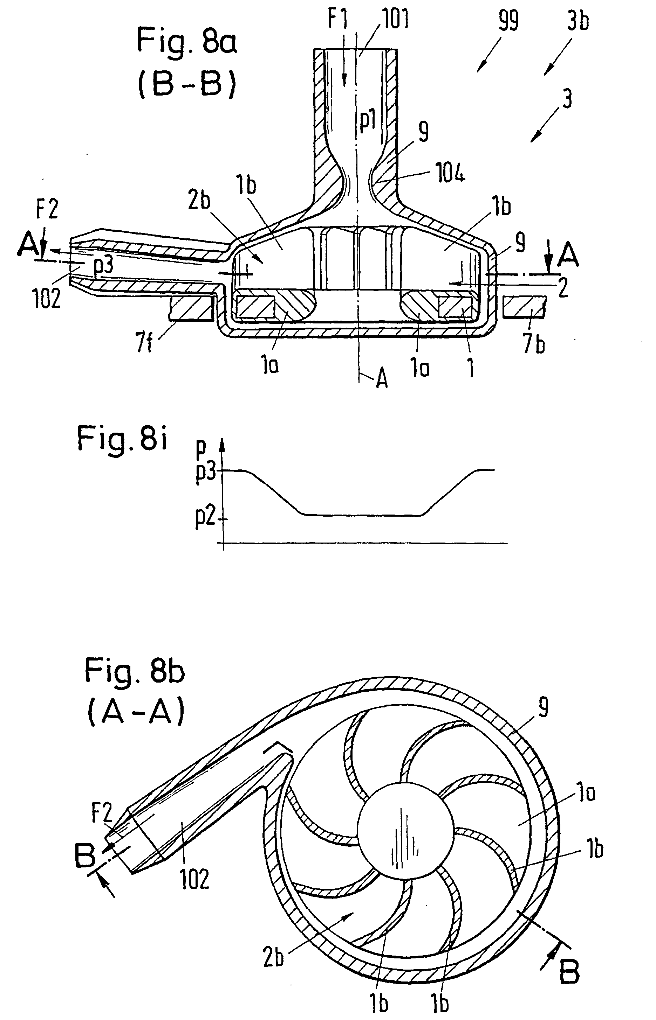

Fig. 8a zeigt einen Längsschnitt durch eine als Zentrifugalpumpe 3b ausgestaltete Rotationspumpe 3. Die Zentrifugalpumpe 3b besteht aus einem Zentrifugalpumpenteil 99, welcher das Gehäuse 9 mit innenliegendem Zentrifugalrotor 2b umfasst, sowie einer Antriebs- und Lagervorrichtung, welche durch die Zähne 7b,7f des Stators 7 angedeutet ist. Der Stator 7 kann wie im Ausführungsbeispiel gemäss Fig. 1a oder Fig. 4a dargestellt ausgebildet sein. Das Gehäuse 9 kann fest oder lösbar mit der Antriebsvorrichtung verbunden sein. In einer vorteilhaften Ausgestaltung der Zentrifugalpumpe 3b ist das Gehäuse 9, wie in Fig. 8a dargestellt, teilweise zwischen die Zähne 7b,7f des Stators 7 einführbar und liegt lose auf der Antriebsvorrichtung auf. Das Gehäuse 9 lässt sich dadurch auf einfache Weise wieder aus dem Stator 7 entfernen und zum Beispiel auswechseln. Wird der Zentrifugalpumpenteil 99 zum Beispiel als eine Blutpumpe verwendet, so kann der Zentrifugalpumpenteil 99 als ein Einmal-Artikel ausgeführt sein. Während somit der Zentrifugalpumpenteil 99 nach jeder Anwendung ersetzt wird, kann die Antriebsvorrichtung beliebig oft verwendet werden zum Antrieb des im Zentrifugalpumpenteil 99 angeordneten Zentrifugalrades 2b. Das Zentrifugalrad 2b ist, wie in den Figuren 1b,1c,1d dargestellt und bereits beschrieben, durch den Stator 7 und die magnetisch wirkenden Kräfte berührungslos schwebend gehalten, sobald der Zentrifugalpumpenteil 99 in der vorgesehenen Lage im Stator 7 angeordnet ist. Durch die passiv und stabilisierend bzw. rückstellend auf die Lage des Rotors 2 wirkenden Kräfte ist dieser bezüglich aller sechs Freiheitsgrade berührungslos im Stator 7 beziehungsweise im Gehäuse 9 lagerbar. Dabei ist zu beachten, dass die rein passiv bewirkten, die Lage des Rotorteils 1 stabilisierend beeinflussenden magnetischen Kräfte, insbesondere die Kraft in axialer Richtung z, von relativ kleinem Betrag ist. Bei grösseren, in z-Richtung Zentrifugalrad 2b angreifenden Kräften, würde dieses bezüglich dem Stator 7 abgehoben beziehungsweise der Rotor 2 würde am Gehäuse 9 des Zentrifugalpumpenteils 99 streifen. Die Figuren 8a bis 8h zeigen verschiedene Ausführungsbeispiele von Zentrifugalpumpenteilen 99 welche alle derart ausgestaltet sind, dass die in z-Richtung am Rotor 2 angreifenden Kräfte vermindert sind, um den Rotor 2 auch während der Förderung eines Fluides berührungslos im Gehäuse 9 zu lagern.

Das Rotorteil 1 kann als ringförmiger Körper in einer Ausführungsform gemäss Fig. 2a, 2c, 2e ausgestaltet sein, oder als scheibenförmiger Körper gemäss einer Ausführungsform von Fig. 10a, 10b; 11a, 11b; 13a,13b. Das Ausführungsbeispiel gemäss Fig. 13a weist ein Permanentmagnet 1p auf, der zwischen ferromagnetischem Material 1q angeordnet ist. Fig. 9 zeigt einen Schnitt durch ein Rotorteil 1 sowie benachbart angeordnete Statorzähne 7b,7f, wobei die sich gegenseitig zugewandten, magnetisch wirksamen Flächen nutenförmige Ausnehmungen aufweisen, um bei einer Auslenkung des Rotorteils 1 in axialer Richtung A eine erhöhte, passiv wirkende Relunktanzkraft in zur Auslenkung entgegengesetzten Richtung zu bewirken.

Fig. 8a zeigt einen Längsschnitt entlang der Linie B-B eines Zentrifugalpumpenteils 99 mit einer in z-Richtung verlaufenden Pumpeneinlassöffnung 101. Der Zentrifugalpumpenteil 99 weist ein Gehäuse 9 mit einem innerhalb des Gehäuses 9 angeordneten Rotor 2 auf. Das Gehäuse 9 ist flüssigkeits- und gasdicht geschlossen und weist eine Pumpeneinlassöffnung 101 sowie eine bezüglich dem Rotor 2 in radialer Richtung verlaufende Pumpenauslassöffnung 102 auf. Der Rotor 2 umfasst ein ringförmig ausgestaltetes, permanentmagnetisiertes Rotorteil 1, wie dies in Fig. 2a dargestellt ist, eine den Rotorteil 1 umgebende Rotorummantelung 1a sowie eine Mehrzahl von über den Umfang des Rotors 2 gleichmässig verteilt angeordnete Schaufeln 1b, welche ein Flügelrad ausbilden.
Das in Richtung F1 einströmende Fluid weist im Bereich der Pumpeneinlassöffnung 101 einen Druck p1 auf. Der Pumpeneinlassöffnung 101 anschliessend ist eine Verengung 104 angeordnet, welche als eine Düse wirkt, sodass das Fluid nach dem Passieren der Düse eine erhöhte Strömungsgeschwindigkeit aufweist. Das Fluid wird in Richtung F2 durch die Pumpenauslassöffnung 102 strömend und unter einem Druck p3 stehend in ein nachfolgendes, nicht dargestelltes Leitungsmittel gefördert. Im Diagramm gemäss Fig. 8i ist der zwischen dem Gehäuse 9 und dem Ringkörper 1a anliegende Druck p des Fluides dargestellt. Dieser Druck erzeugt eine in z-Richtung weisende Kraft auf den Rotor 2, welche möglichst gering gehalten werden sollte. Diese in z-Richtung wirkende Kraft wird im Ausführungsbeispiel gemäss Fig. 8a gering gehalten indem der Rotor 2 als ein Ringkörper ausgebildet ist, sodass im Bereich der Ausnehmung des Ringkörpers keine in z-Richtung wirkende Kraft auf den Rotor 2 erzeugt wird. Durch diese Massnahme entsteht während dem Pumpbetrieb eine nur geringe, in z-Richtung auf den Rotor 2 wirkende Kraft auf. Fig. 8b zeigt einen Schnitt durch Fig. 8a entlang der Linie A-A mit Gehäuse 9, Rotor 2b, Ringkörper 1a und Schaufeln 1b beziehungsweise Flügeln 1b. Der Zentrifugalrotor 2b ist ringförmig ausgestaltet.

Fig. 8c zeigt einen Längsschnitt und Fig. 8d einen Querschnitt durch ein weiteres Ausführungsbeispiel eines Zentrifugalpumpenteils 99. Zusätzlich zur Ausgestaltung gemäss Fig. 8a weist der Zentrifugalrotor 2 eine kreisförmig ausgestaltete Prallplatte 1k auf, welche über in radialer Richtung verlaufende Stege 1l mit dem Ringkörper 1a verbunden ist. Die Prallplatte 1k ist senkrecht zur Verlaufsrichtung der Pumpeneinlassöffung 101 angeordnet, derart, dass ein wesentlicher Teil des in Richtung F1 strömenden Fluides in Richtung F3 strömend auf die Prallplatte 1k trifft und sich anschliessend in eine Vielzahl von Teilströmen F4 aufteilt. Diese Prallplatte 1k bewirkt insbesondere bei grossen Fördermengen des Fluides eine in Richtung F1 wirkende Kraft auf den Rotor 2.

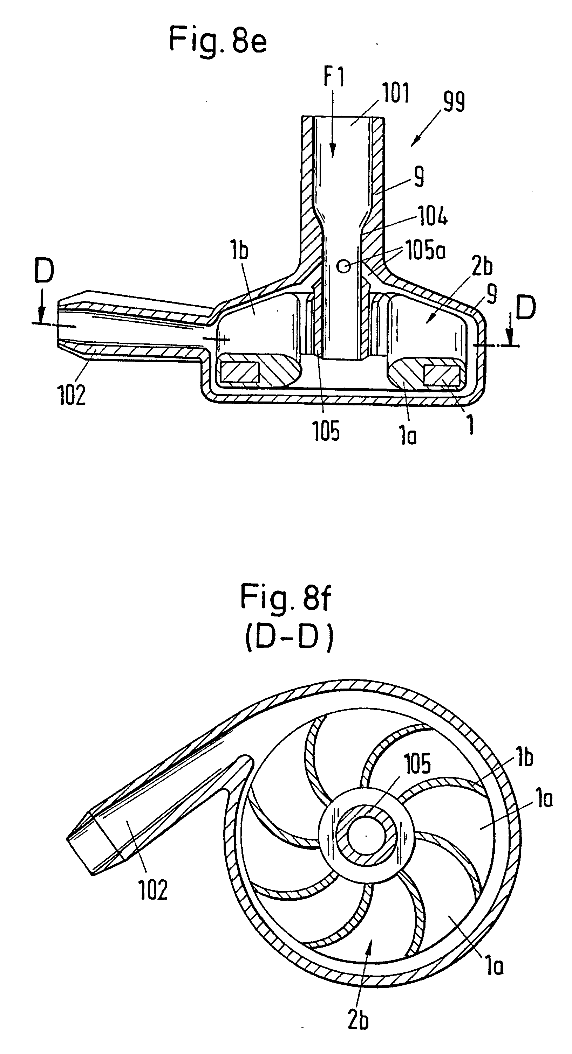

Fig. 8e zeigt einen Längsschnitt und Fig. 8f einen Querschnitt durch ein weiteres Ausführungsbeispiel eines Zentrifugalpumpenteils 99. Zusätzlich zur Ausgestaltung gemäss Fig. 8a weist der dargestellte Zentrifugalpumpenteil 99 einen in axialer Richtung, bzw. in z-Richtung verlaufenden, hohlzylinderförmigen Rohrfortsatz 105 auf, welcher anschliessen an die Pumpeneinlassöffnung 101 angeordnet ist. Dadurch wird das in Richtung F1 strömende Fluid dirket in das Zentrum des Ringkörpers 1a geleitet. Die Länge des Rohrfortsatzes 105 kann unterschiedlich ausgestaltet sein. Der statische Druck des Fluides im Bereich der kreisförmigen Öffnung im Zentrum des Ringkörpers 1a ist abhängig von der Lage der Austrittsöffnung des Rohrfortsatzes 105. Je nach Länge des Rohrfortsatzes 105 kann somit die durch das einströmende Fluid in F1-Richtung auf den Rotor 2 bewirkte Kraft bestimmt werden. Damit sich beim Füllen des Zentrifugalpumpenteils 99 mit einem Fluid keine Luftblasen bilden weist der Rohrfortsatz 105 Durchbrechungen 105a auf, die derart angeordnet sind, dass während dem Einfüllen eines Fluides über die Pumpenauslassöffnung 102 der Flüssigkeitsspiegel innerhalb des Gehäuses 9 ansteigt, und sich im Rotorinnenraum bildende Luftblasen über die Durchbrechungen 105a zur Pumpeneinlassöffnung 101 hin entweichen. Fig. 8f zeigt in einer Schnittdarstellung entlang der Linie D-D gemäss Fig. 8e den ringförmig ausgestalteten Rotor 2 mit dem im Zentrum angeordneten Rohrfortsatz 105.

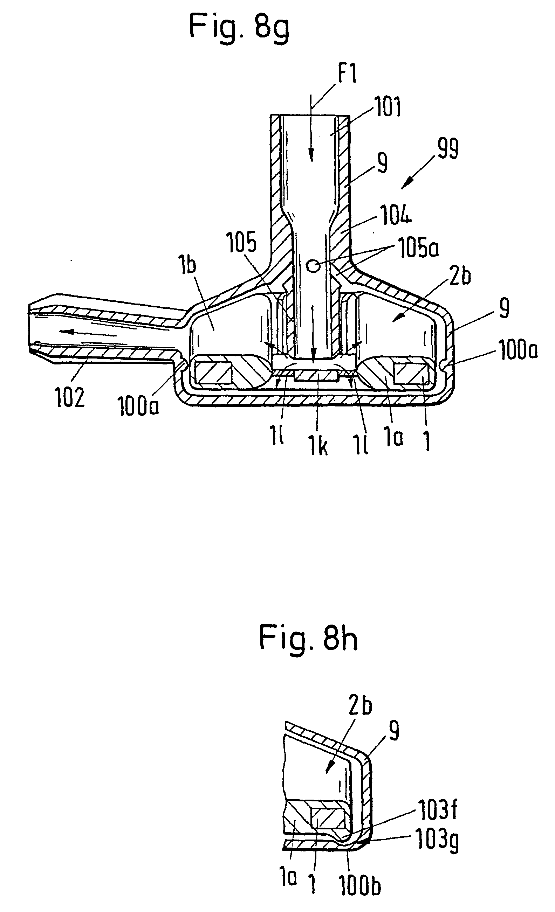

Fig. 8g zeigt einen Längsschnitt durch ein weiteres Ausführungsbeispiel eines Zentrifugalpumpenteils 99. Der Rotor 2 weist, wie in Fig. 8c ausführlich beschrieben, eine Prallplatte 1k auf, welche über Stege 1l mit dem Ringkörper 1a verbunden ist. Zudem weist des Zentrifugalpumpenteil 99, wie in Fig. 8e ausführlich beschrieben, einen hohlzylinderförmigen Rohrfortsatz 105 auf. Diese beiden Massnahmen bewirken während dem Fördern des Fluides eine in Richtung der Flussrichtung F1 wirkende Kraft auf den Rotor 2. Diese Kraft kann durch eine Vielzahl weiterer Massnahmen mit entsprechender Ausgestaltung strömungsbestimmender Komponeten erzielt werden, um das Strömungsverhalten des Fluides derart zu beeinflussen, dass eine bezüglich dem Rotor 2 in axialer Richtung wirkende Kraft auf diesen erzeugt wird. So kann beispielsweise an der Innenwand des Gehäuses 9 ein über den gesamten Umfang verlaufendes, vorstehendes Teil 100a angeordnet sein, um den Druck des Fluides zwischen dem Boden des Gehäuses 9 und dem Rotor 2 zu reduzieren. Fig. 8h zeigt in einer Detailansicht eine weitere Massnahme zur Reduzierung dieses Druckes indem am Boden des Gehäuses 9 eine über den gesamten Umfang verlaufende Vertiefung 100b angeordnet ist, und der Ringkörper 1a ein der Vertiefung 110b entsprechend angepasst ausgestaltetes, über den gesamten Umfang verlaufendes vorstehendes Teil 103f aufweist. Der Dichtungsspalt 103g zwischen der Vertiefung 100b am Gehäuse 9 und dem vorstehenden Teil 103f im Rotor 2 bildet einen Fliesswiderstand für das den Rotor 2 umströmende Fluid.

Der Fliesswiderstand ändert sich angenähert proportional zur Breite des Dichtungsspaltes 103g. Falls der Rotor nach oben ausgelenkt wird, so wird der Fliesswiderstand kleiner, wodurch der Druck unter dem Rotor sinkt und der Rotor in seine ursprüngliche Lage zurückkehrt. Somit ergibt sich in axialer Richtung eine selbstregulierende Rotorpositionierung.

Der Kerngedanke, die den Fluss des Fluides bestimmenden Komponenten des Zentrifugalpumpenteils 99 derart auszugestalten, dass während dem Fliessen des Fluides eine in axialer Richtung auf den Zentrifugalrotor 2b wirkende Kraft erzeugt wird, kann durch eine Vielzahl hydrodynamisch wirkender Massnahmen erzielt werden, so dass die in den Figuren 8a bis 8i dargestellten Ausführungen nur Beispiele aus einer Vielzahl möglicher Ausführungsformen darstellen.

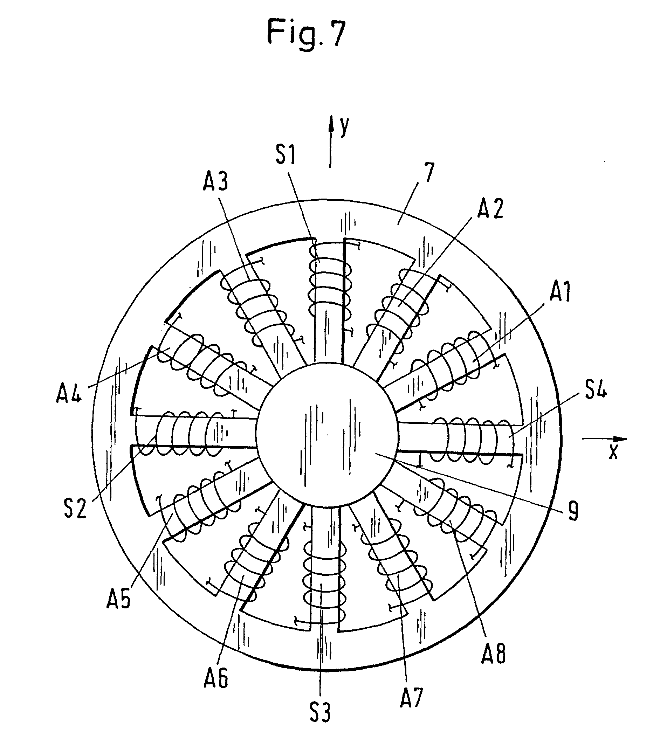

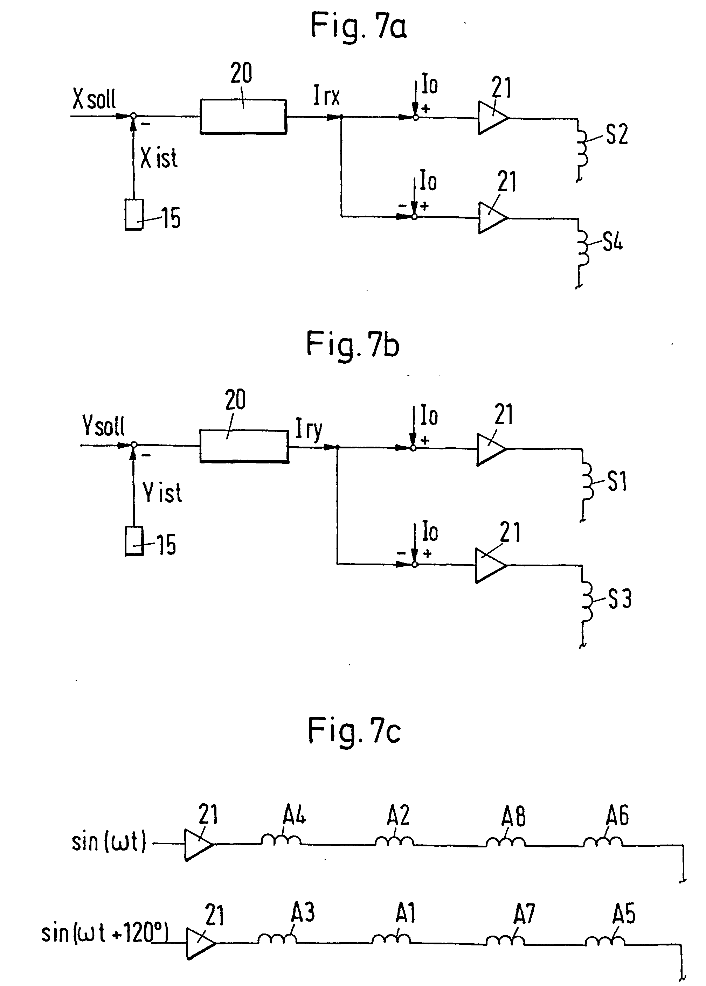

Fig. 8k zeigt schematisch in einer perspektivischen Ansicht eine Zentrifugalpumpe 3b mit in den Stator 7 eingesetztem Zentrifugalpumpenteil 99. Der Stator 7 ist in diesem Ausführungsbeispiel als ein sogenannter Tempelmotor ausgestaltet, indem die die Zähne 7a,7b,7c,7d,7e,7f,7g,7h ausbildenden Flusseisen 7y L-förmig ausgebildet sind, und die Spulen 8a,8b,8c,8d,8e,8f,8g,8h im vertikal verlaufenden Abschnitt des Flusseisens 7y angeordnet sind. Alle Zähne 7a,7b,7c,7d,7e,7f,7g,7h sind über das scheibenförmig ausgestaltete Rückschlusseisen 7z miteinander magnetisch gekoppelt. Die Ausführungsformen gemäss Fig. 1a und Fig. 8k sind auf eine identische Weise ansteuerbar und haben dieselbe Wirkung auf den magnetisch wirksamen Rotorteil 1. Ein Vorteil des Stators 7 in der Ausführungsform gemäss Fig. 8k ist darin zu sehen, dass das Zentrifugalpumpenteil 99, als ein auswechselbares Teil ausgestaltet, auf besonders einfache Weise in den Stator 7 einführbar und wieder entfernbar ist. Die Zähne 7a,7b,7c,7d,7e,7f,7g,7h und die entsprechenden Wicklungen 8a,8b,8c,8d,8e,8f,8g,8h des Stators 7 können auf unterschiedlichste Weise angeordnet sein, so auch gemäss einer wie in den Fig. 4a, 4b, 4d, 7 dargestellten Anordnung, wobei der magnetische Rückschluss immer über das scheibenförmige Rückschlusseisen 7z erfolgt. Die Wicklungen können im horizontal oder vertikal verlaufenden Teilabschnitt des Flusseisens 7y angeordnet sein.

Der in Fig. 1a dargestellte lagerlose Motor, umfassend den Stator 7, den Rotorteil 1 sowie die Wicklungen 8b,8c,8d,8e,8f, kann auch einen Stator 7 gemäss der in Fig. 6c dargestellten Ausführungsform aufweisen. Der Stator gemäss Fig. 6c weist in axialer Richtung A verlaufende Statornuten mit eingelegten Wicklungen 8 auf. Die Zähne 7a, 7b,... sind zwischen den Statornuten verlaufend ausgebildet. Wird der Stator 7 gemäss Fig. 1a durch den Stator 7 gemäss Fig. 6c ersetzt, so kann dieser Stator 7 beispielsweise eine Antriebswicklung WA, wie in Fig. 4h dargestellt, und eine Steuerwicklung WS, wie in Fig. 4i dargestellt aufweisen. Fig. 4h zeigt das Wickelschema der Antriebswicklung WA, welche als eine dreiphasige, zweipolige Einschichtwicklung in den 36 Nuten aufweisenden Stator 7 eingelegt ist. Fig. 4i zeigt das Wickelschema der Steuerwicklung WS, welche als eine dreiphasige, vierpolige Einschichtwicklung in die 36 Nuten desselben Stators 7 eingelegt ist. Ein derartiger Stator 7 mit einem Rotor gemäss Fig. 1a ist mit zwei Drehstromstellern, je einen für die Antriebswicklung WA und die Steuerwicklung WS antreibbar. Der Rotorteil 1 kann beim vorhin dargestellten Stator 7 auch ohne permanentmagnetisiert zu sein als ein Käfigläufer ausgestaltet sein oder eine kurzgeschlossene Wicklung aufweisen. Zum Antrieb des Rotorteils 1 wird durch das am Stator 7 erzeugte Drehfeld ein Stromfluss im Rotorteil 1 induziert, sodass auf den Rotorteil 1, vergleichbar dem Antriebsprinzip eines Induktionsmotors, ein antreibendes Drehmoment bewirkt wird.

Fig. 7 zeigt ein weiteres Ausführungsbeispiel eines Stators 7 mit zwölf in radialer Richtung verlaufend um das Gehäuse 9 angeordneten Zähnen. Die Steuerwicklung WS besteht aus den vier orthogonal angeordneten Wicklungen S1,S2,S3,S4 und die Antriebswicklung WA setzt sich zusammen aus den Wicklungen A1,A2,A3,A4,A5,A6,A7,A8. Dieser Stator 7 ist geeignet ein magnetisch wirksames Rotorteil 1 gemäss einer in den Fig. 2a bis 2h, oder in den Fig. 10a,10b,11a,11b,13a,13b dargestellten Ausführungsformen anzutreiben und berührungslos zu lagern. Fig. 7a zeigt ein Ausführungsbeispiel einer Ansteuervorrichtung für einen Stator 7 gemäss Fig. 7 zur Regelung des Lage des Rotors 2 in x-Richtung. Mit einem Positionsmesssensor 15 wird die gegenwärtige Lage xist des Rotors 2 erfasst, dieser Wert mit einem Sollwert Xsoll verglichen, und der Differenzwert einer Regelvorrichtung 20 zugeführt, welche eine Stellgrösse Irx berechnet. Die Spule S4 sowie die Spule S2 werden über je einen Verstärker 21 mit einem Grundstrom Io gespeist. Die Stellgrösse Irx wird dem Grundstrom Io überlagert, wobei für den Gesamtstrom der Spule S2 die Summe und für den Gesamtstrom der Spule S4 die Differenz gebildet wird, sodass eine entsprechende in x-Richtung verlaufende Kraft auf den Rotor 2 ausgeübt wird. Fig. 7b zeigt dieselbe Ansteuervorrichtung zur Regelung der Lage des Rotors 2 in y-Richtung. Die Regeldifferenz ysoll minus yist wird der Regelvorrichtung 20 zugeführt, welche eine Stellgrösse Iry berechnet, welche den Spulen S1 und S3 zugeführt wird, um eine entsprechende in y-Richtung wirkende Kraft auf den Rotor 2 zu bewirken. Fig. 7c zeigt die Ansteuerung der das Drehmoment auf den Rotor 2 beziehungsweise der das magnetische Drehfeld bewirkenden Spulen A1,A2,A3,A4,A5,A6,A7,A8. Die Spulen sind mit zwei Phasen eines Dreiphasensystems verbunden, wobei die erste Phase eine Spannung sin(ωt) erzeugt und die zweite Phase eine Spannung sin(ωt + 120°), d.h. mit einer Phasenverschiebung von 120 Grad bezüglich der ersten Phase. Die Spulen A4,A2,A8,A6 sind in Serie geschaltet und werden von einem gemeinsamen Verstärker 21 mit einer Spannung sin(ωt) der ersten Phase angesteuert. Die Spulen A3,A1,A7,A5 sind ebenfalls in Serie geschaltet und werden von einem gemeinsamen Verstärker 21 mit einer Spannung sin (ωt + 120°) der zweiten Phase angesteuert. Durch diese Ansteuerung wird im Stator 7 ein magnetisches Drehfeld erzeugt, welches ein in Umfangsrichtung auf den Rotor 2 wirkendes Drehmoment erzeugt, um diesen anzutreiben.

Fig. 14a zeigt einen Längsschnitt durch ein weiteres Ausführungsbeispiel eines Zentrifugalpumpenteils 99, welches, ansonst gleich ausgestaltet wie das Ausführungsbeispiel gemäss Fig. 8a, zusätzlich ringförmig ausgestaltete Permanentmagnete 80a,80b,80c,80d aufweist, um eine in Verlaufsrichtung der Achse A wirkende Axialschubkompensation zu bewirken, wobei der Rotorteil 1 zusätzlich eine Deckplatte 81 umfasst, mit welchem ein Permanentmagnet 80c fest verbunden ist. In der Antriebsvorrichtung, umfassend den Stator 7 mit Zähnen 7a,7b,7c,7d,7e,7f,7g,7h, ist zusätzlich ein ringförmiger, in axialer Richtung A polarisierter Permanentmagnet 80a angeordnet. Beim Einführen des Zentrifugalpumpenteils 99 in den Stator 7 kommt das Gehäuse 9 über oder auf den Permanentmagnet 80a zu stehen. Das Rotorteil 1 des Zentrifugalpumpenteils 99 weist ebenfalls ein ringförmiger, in axialer Richtung A polarisierter Permanentmagent 80b auf, welcher in zum Permanentmagent 80a entgegengesetzter Richtung polarisiert ist, sodass zwischen den Permanentmagneten 80a und 80b eine abstossende, permanentmagnetisch bewirkte Kraft auftritt. Die beiden ringförmigen Permanentmagnete 80a,80b sind bezüglich deren Durchmesser derart gegenseitig angepasst ausgestaltet, dass deren magnetisch wirksamen Stirnflächen bei eingesetztem Zentrifugalpumpenteil 99 übereinander zu liegen kommen. Desgleichen weist der im oberen Bereich des Rotorteils 1 durch die Deckplatte 81 fest mit dem Rotorteil 1 verbundene Permanentmagnet 80c ein entsprechend angepasst ausgestalteter, ausserhalb des Zentrifugalpumpenteils 99 angeordneter und auf diesem aufliegenden ringförmigen Permanentmagentring 80d auf. Diese beiden ringförmigen Permanentmagnete 80c, 80d sind in entgegengesetzter Richtung polarisiert und über daher eine sich gegenseitig abstossende, permanentmagnetisch bewirkte Kraft aufeinander aus. In einer vorteilhaften Ausgestaltung sind die innerhalb des Zentrifugalpumpenteils 99 angeordneten Permanentmagnete 80b, 80c kleiner oder wesentlich kleiner ausgestaltet als die ausserhalb liegenden Permanentmagnete 80a und 80d. Diese Anordnung ist insbesondere vorteilhaft wenn der Zentrifugalpumpenteil 99 als ein Einmalartikel ausgestaltet ist, zum Beispiel als eine Blutpumpe, welche nach deren einmaligen Verwendung vernichtet wird.

Fig. 14b zeigt einen Längsschnitt durch ein weiteres Ausführungsbeispiel einer Axialpumpe 3a, welche, ansonst gleich ausgestaltet wie das Ausführungsbeispiel gemäss Fig. 5a, zusätzlich ringförmig ausgestaltete Permanentmagnete 80a,80b,80c,80d,80e,80f aufweist, um eine in Verlaufsrichtung der Achse A wirkende Axialschubkompensation zu bewirken. An beiden Seiten des Rotors 2 ist an dessen Endbereich ein ringförmiger, in axialer Richtung A polarisierter Permanentmagnet 80b, 80c eingegossen. Im feststehenden Gehäuse 9 sind weitere ringförmige Permanentmagnete 80a,80d,80e,80f derart angeordnet und derart polarisiert, dass eine beidseitig in axialer Richtung A wirkende, permanentmagnetisch erzeugte, abstossende Kraft auf den Rotor 2 erzeugt wird. Durch diese Anordnung der Permanentmagnete 80a,80b,80c,80d,80e,80f wird eine in axialer Richtung A wirksame Schubkompensation auf den Rotor 2 bewirkt. In beiden dargestellten Ausführungsbeispielen Fig. 14a und Fig. 14b gibt es eine Vielzahl von sinnvollen Anordnungsmöglichkeiten der Permanentmagnete 80a,80b,80d,80c,80e,80f, um eine durch permanentmagnetische Kräfte bewirkte Axialschubkompensation zu erzeugen. So könnte beispielsweise die Axialpumpe 3a gemäss Ausführungsbeispiel 14b auch nur die links angeordneten Permanentmagnete 80a,80b aufweisen, um eine in axialer Richtung A wirkende Schubkompensation zu erzeugen. Ebenso könnte die Anordnung gemäss Fig. 14a nur die im unteren Bereich angeordneten Permanentmagnete 80a,80b aufweisen, um eine in axialer Richtung A wirkende Schubkompensation zu erzeugen. Die ringförmig verlaufenden Permanentmagnete 80a,80b,80c,80d,80e,80f können beispielsweise auch aus einer Mehrzahl einzelner Segmente zusammengesetzt sein. Die Permanentmagnete 80a,80b,80c,80d,80e,80f können auch in radialer Richtung polarisiert sein und vorzugsweise leicht versetzt übereinander angeordnet sein, um eine permanentmagnetisch erzeugte, in axialer Richtung wirkende Kraft zu erzeugen. Gewisse Permanentmagnete 80a,80b können auch radial magnetisiert und die restlichen Permanentmagnete 80c,80d,80e,80f axial polarisiert sein, wobei die Permanentmagnete derart gegenseitig wirksam angeordnet sind, dass eine in axialer Richtung wirksame Kraft erzeugt wird.

## Patentansprüche

1. Rotationspumpe (3) umfassend ein Gehäuse (9) und einen innerhalb des Gehäuses (9) angeordneten Rotor (2) mit Schaufeln (1b), bei welcher Rotationspumpe der Rotor (2) ein passives, magnetisch wirksames Rotorteil (1) umfasst und das Gehäuse (9) von einem elektrische Wicklungen (8a,8b,8c,8d,8e,8f,8g,8h) und Zähne (7a,7b,7c,7d,7e,7f,7g,7h) aufweisenden Stator (7) umgeben ist, wobei der Stator (7) und der Rotorteil (1) einen lagerlosen Motor bilden, **dadurch gekennzeichnet, dass** der Rotorteil (1) mittels der elektrischen Wicklungen (8a,8b,8c,8d,8e,8f,8g,8h) bezüglich dreier Freiheitsgrade aktiv ansteuerbar und antreibbar ist, und dass der Rotorteil (1) und die Zähne (7a,7b,7c,7d,7e,7f,7g,7h) des Stators (7) derart geometrisch gegenseitig angepasst ausgestaltet und zueinander angeordnet sind, dass der Rotorteil (1) bezüglich dreier weiterer, nicht aktiv ansteuerbarer Freiheitsgrade durch passiv wirkende Reluktanzkräfte im Stator (7) haltbar ist, um den Rotor (2) innerhalb des Gehäuses (9) antreibbar und berührungslos schwebend anzuordnen.

2. Rotationspumpe nach Anspruch 1, **dadurch gekennzeichnet, dass** der Rotorteil (1) eine Drehachse (A) aufweist, und dass bezüglich dieser axialen Richtung (A) der Rotorteil (1) eine Höhe (HR) und die Zähne (7a,7b,7c,7d,7e,7f,7g,7h) eine Höhe (HS) aufweisen, dass die Höhe (HR) des Rotorteil (1) gleich oder ungefähr gleich der Höhe (HS) der Zähne (7a,7b,7c,7d,7e,7f,7g,7h) ausgestaltet ist, und dass der Durchmesser (DR) des Rotorteils (1) zumindest doppelt so gross ist wie dessen Höhe (HR).

3. Rotationspumpe nach einem der Ansprüche 1 oder 2, **dadurch gekennzeichnet, dass** der Rotorteil (1) als ein scheibenförmiger oder ringförmiger Körper ausgestaltet ist.

4. Rotationspumpe nach einem der Ansprüche 1 bis 3, **dadurch gekennzeichnet, dass** der Rotorteil (1) als ein sternförmiger Körper ausgestaltet ist.

5. Rotationspumpe nach einem der Ansprüche 1 bis 4, **dadurch gekennzeichnet, dass** der Rotorteil (1) aus einem Permanentmagnet besteht oder einen Permanentmagnet umfasst.

6. Rotationspumpe nach einem der Ansprüche 1 bis 5, **dadurch gekennzeichnet, dass** der Rotorteil (1) eine kurzgeschlossene Wicklung oder einen Käfigläufer aufweist.

7. Rotationspumpe nach einem der Ansprüche 1 bis 6, **dadurch gekennzeichnet, dass** der Rotorteil (1) von einer Rotorummantelung (1a), bestehend aus einem nicht ferromagnetischen Material, insbesondere Kunststoff, Metall, Keramik oder einem biokompatiblen Werkstoff, umschlossen ist.

8. Rotationspumpe nach einem der Ansprüche 1 bis 7, **dadurch gekennzeichnet, dass** der Rotorteil (1) sowie der Stator (7) derart gegenseitig ausgestaltet sind und die elektrischen Wicklungen (8a,8b,8c,8d,8e,8f,8g,8h) derart von einer Ansteuervorrichtung (40) ansteuerbar sind, dass der Rotorteil (1) des lagerlosen Motors (4) gemäss dem Funktionsprinzip eines Reluktanzmotors, eines Synchronmotors oder eines Induktionsmotors antreibbar ist.

9. Rotationspumpe nach Anspruch 8, **dadurch gekennzeichnet, dass** der Stator (7) eine Antriebswicklung (WA) mit einer Polpaarzahl p und eine Steuerwicklung (WS) mit einer Polpaarzahl p+1 oder p-1 aufweist.

10. Rotationspumpe nach einem der Ansprüche 1 bis 9, **dadurch gekennzeichnet, dass** in axialer Richtung (A) neben dem Rotor (2) zumindest auf der einen Seite eine Notlauflagerstelle (9b) angeordnet ist.

11. Rotationspumpe nach einem der Ansprüche 1 bis 11, **dadurch gekennzeichnet, dass** der Rotor (2) und ein Teilabschnitt der Innenwand des Gehäuses (9) derart gegenseitig verlaufend ausgebildet sind, dass ein zumindest in axialer Richtung (A) wirkendes hydrodynamisches Lager ausgebildet ist.

12. Rotationspumpe nach einem der Ansprüche 1 bis 11, **dadurch gekennzeichnet, dass** der Rotor (2) an der dem Gehäuse (9) zugewandten Oberfläche zumindest eine wendelförmig verlaufende Rille aufweist, zur Ausbildung eines Spurlagers.

13. Rotationspumpe nach einem der Ansprüche 1 bis 12, **dadurch gekennzeichnet, dass** die Rotationspumpe (3) als eine Axialpumpe (3a) ausgebildet ist, und dass der Rotorteil (1) im Drehzentrum eine Ausnehmung aufweist, in welcher die Schaufeln (1b) des als Axialrad (2a) ausgestalteten Rotors (2) angeordnet sind.

14. Rotationspumpe nach Anspruch 13, **dadurch gekennzeichnet, dass** die Schaufeln (1b) in axialer Richtung (A) über die Rotorummantelung (1a) vorstehend verlaufend angeordnet sind.

15. Rotationspumpe nach einem der Ansprüche 1 bis 12, **dadurch gekennzeichnet, dass** die Rotationspumpe (3) als eine Axialpumpe (3a) ausgebildet ist, dass der Rotor (2) nur aus dem Rotorteil (1) besteht, und dass der Rotorteil (1) derart verlaufend ausgestaltet ist, dass dieser zugleich die Schaufeln (1b) des Axialrades (2a) ausbildet.

16. Rotationspumpe nach einem der Ansprüche 1 bis 12, **dadurch gekennzeichnet, dass** die Rotationspumpe (3) als eine Zentrifugalpumpe (3b) ausgebildet ist, und dass der Rotor (2) als Zentrifugalrad (2b) ausgebildet ist und in axialer Richtung (A) zumindest auf einer Seite Schaufeln (1b) aufweist.

17. Rotationspumpe nach Anspruch 16, **dadurch gekennzeichnet, dass** die Innenwandung des Gehäuses (9) und/oder das Zentrifugalrad (2b) strömungsbeeinflussende Teilelemente (100a, 100b, 104, 105, 103f, 11, 1k) aufweist, um während einem Pumpbetrieb eine im wesentlichen in axialer Richtung (A) wirkende Kraft auf das Zentrifugalrad (2b) zu bewirken.

18. Rotationspumpe nach einem der Ansprüche 16 oder 17, **dadurch gekennzeichnet, dass** das Gehäuse (9) mit innenliegendem Zentrifugalrad (2b) zumindest teilweise in den Stator (7) einführbar und wieder entfernbar ausgestaltet ist.

19. Rotationspumpe nach einem der Ansprüche 1 bis 18, **dadurch gekennzeichnet, dass** der Rotorteil (1) zumindest ein ringförmig verlaufender Permanentmagnet (80b,80c) umfasst, und dass die Rotationspumpe (3) zumindest ein weiterer, fest angeordneter Permanentmagnet (80a,80d) umfasst, und dass die Permanentmagnete (80b,80c,80a,80d) derart gegenseitig wirksam polarisiert und angeordnet sind, dass eine in axialer Richtung (A) wirksame, abstossende Kraft auf den Rotorteil (1) entsteht.

20. Blutpumpe mit einer Rotationspumpe (3, 3a, 3b) nach einem der Ansprüche 1 bis 19.

## Claims

1. Rotary pump (3) comprising a housing (9) and a rotor (2) with blades (1b) arranged inside housing (9), in which rotor pump the rotor (2) comprises a passive magnetically effective rotor part (1), and the housing (9) is surrounded by a stator (7) that has electrical windings (8a, 8b, 8c, 8d, 8e, 8f, 8g, 8h) and teeth (7a, 7b, 7c, 7d, 7e, 7f, 7g, and 7h), with the stator (7) and the rotor part (1) forming a bearingless motor, **characterized in that** the rotor part (1) is actively controllable and drivable by means of electrical windings (8a, 8b, 8c, 8d, 8e, 8f, 8g, and 8h) with respect to three degrees of freedom; and **in that** the rotor part (1) and the teeth (7a, 7b, 7c, 7d, 7e, 7f, 7g, and 7h) of the stator (7) are designed geometrically adapated with respect to one another and arranged with respect to one another such that the rotor part (1) can be held in the stator (7) with respect to three additional non-actively-controllable degrees of freedom by passively acting reluctance forces in order to locate the rotor (2) inside the housing (9) in a drivable and floating manner with zero contact.

2. Rotary pump according to claim 1, **characterized in that** the rotor part (1) has a rotation axis (A) and **in that** the rotor part (1) has a height (HR) and the teeth (7a, 7b, 7c, 7d, 7e, 7f, 7g, and 7h) have a height (HS) with respect to this axial direction (A); **in that** the height (HR) of the rotor part (1) is equal to or approximately equal to the height (HS) of the teeth (7a, 7b, 7c, 7d, 7e, 7f, 7g, and 7h); and **in that** the diameter (DR) of the rotor part (1) is at least twice as great as its height (HR).

3. Rotary pump according to one of claims 1 or 2, **characterized in that** the rotor part (1) is designed as a disk-shaped or annular body.

4. Rotary pump according to one of claims 1 to 3, **characterized in that** the rotor part (1) is designed as a star-shaped body.

5. Rotary pump according to one of claims 1 to 4, **characterized in that** the rotor part (1) consists of a permanent magnet or comprises a permanent magnet.

6. Rotary pump according to one of claims 1 to 5, **characterized in that** the rotor part (1) comprises a short-circuited winding or a cage rotor.

7. Rotary pump according to one of claims 1 to 6, **characterized in that** the rotor part (1) is surrounded by a rotor jacket (1a) consisting of a non-ferromagnetic material, in particular plastic, metal, ceramic, or a biocompatible material.

8. Rotary pump according to one of claims 1 to 7, **characterized in that** the rotor part (1) as well as the stator (7) are designed relative to one another and the electrical windings (8a, 8b, 8c, 8d, 8e, 8f, 8g, and 8h) are controllable by a control device (40) such that the rotor part (1) of bearingless motor (4) is drivable according to the functional principle of a reluctance motor, a synchronous motor, or an induction motor.

9. Rotary pump according to claim 8, **characterized in that** stator (7) has a drive winding (WA) with a pole pair number p and a control winding (WS) with a pole pair number p+1 or p-1.

10. Rotary pump according to one of claims 1 to 9, **characterized in that** an emergency bearing location (9b) is located in the axial direction (A) adjacent the rotor (2) on at least one side.

11. Rotary pump according to one of claims 1 to 11, **characterized in that** the rotor (2) and a partial section of the inside wall of the housing (9) are designed with respect to one another in such fashion that a hydrodynamic bearing is formed that acts at least in the axial direction (A).

12. Rotary pump according to one of claims 1 to 11, **characterized in that** the rotor (2), on the surface facing the housing (9), has at least one helical groove for forming a track bearing.

13. Rotary pump according to one of claims 1 to 12, **characterized in that** the rotary pump (3) is designed as an axial pump (3a) and **in that** the rotor part (1) has a recess at its center of rotation in which blades (1b) of rotor (2) designed as axial impellers (2a) are located.

14. Rotary pump according to claim 13, **characterized in that** the blades (1b) are arranged projecting in the axial direction (A) beyond the rotor jacket (1a).

15. Rotary pump according to one of claims 1 to 12, **characterized in that** the rotary pump (3) is designed as an axial pump (3a); **in that** the rotor (2) consists only of rotor part (1); and **in that** rotor part (1) is so designed that it simultaneously forms the blades (1b) of an axial impeller (2a).

16. Rotary pump according to one of claims 1 to 12, **characterized in that** the rotary pump (3) is designed as a centrifugal pump (3b); and **in that** the rotor (2) is designed as a centrifugal impeller (2b) and has blades (1b) extending in the axial direction (A) on at least one side.

17. Rotary pump according to claim 16, **characterized in that** the inside wall of housing (9) and/or the centrifugal impeller (2b) comprise flow-influencing partial elements (100a, 100b, 104, 105, 103f, 11, 1k) in order to exert a force on centrifugal impeller (2b) during pump operation that acts essentially in the axial direction (A).

18. Rotary pump according to one of claims 16 or 17, **characterized in that** the housing (9) with the internal centrifugal impeller (2b) can be inserted at least partially into the stator (7) and can be removed again.

19. Rotary pump according to one of claims 1 to 18, **characterized in that** the rotor part (1) comprises at least one annular permanent magnet (80b,80c); **in that** the rotary pump (3) comprises at least one further fixedly arranged permanent magnet (80a,80d); and **in that** the permanent magnets (80a,80b,80c,80d) are arranged and actively polarized with respect to one another such that a repulsive force acting on the rotor part (1) in the axial direction (A) is generated.

20. Blood pump, comprising a rotary pump (3, 3a, 3b) according to one of claims 1 to 19.

## Revendications

1. Pompe rotative (1) comportant un boîtier (9) et un rotor (2) avec des aubes (1b) disposé à l'intérieur du boîtier (9), où dans cette pompe rotative, le rotor (2) comprend une partie de rotor passive (1), à effet magnétique et le boîtier (9) est entouré par un stator (7) présentant des enroulements électriques (8a, 8b, 8c, 8d, 8e, 8f, 8g, 8h) et des dents (7a, 7b, 7c, 7d, 7e, 7f, 7g, 7h), où le stator (7) et la partie de rotor (1) constituent un moteur sans palier, **caractérisée en ce que** la partie de rotor (1) peut être commandée et entraînée activement au moyen des enroulements électriques (8a, 8b, 8c, 8d, 8e, 8f, 8g, 8h) concernant trois degrés de liberté, et **en ce que** la partie de rotor (1) et les dents (7a, 7b, 7c, 7d, 7e, 7f, 7g, 7h) du stator (7) sont configurées pour être adaptées géométriquement mutuellement et sont disposées les unes par rapport aux autres de façon que la partie de rotor (7) peut être maintenue relativement à trois autres degrés de liberté, ne pouvant pas être commandés activement, par des forces de réluctance à effet passif dans le stator (7) pour disposer le rotor (2) à l'intérieur du boîtier (9) de façon qu'il puisse être entraîné et être suspendu sans contact.

2. Pompe rotative selon la revendication 1, **caractérisée en ce que** la partie de rotor (1) présente un axe de rotation (A), et **en ce que** relativement à cette direction axiale (A) la partie de rotor (1) présente une hauteur (HR) et les dents (7a, 7b, 7c, 7d, 7e, 7f, 7g, 7h) une hauteur (HS), **en ce que** la hauteur (HR) de la partie de rotor (1) est réalisée pour être égale ou approximativement égale à la hauteur (HS) des dents (7a, 7b, 7c, 7d, 7e, 7f, 7g, 7h), et **en ce que** le diamètre (DR) de la partie de rotor (1) représente au moins le double de la hauteur (HR) de celle-ci.

3. Pompe rotative selon l'une des revendications 1 ou 2, **caractérisée en ce que** la partie de rotor (1) est réalisée comme corps en forme de disque ou en forme d'anneau.

4. Pompe rotative selon l'une des revendications 1 à 3, **caractérisée en ce que** la partie de rotor (1) est réalisée comme corps en forme d'étoile.

5. Pompe rotative selon l'une des revendications 1 à 4, **caractérisée en ce que** la partie de rotor (1) est constituée d'un aimant permanent ou comprend un aimant permanent.

6. Pompe rotative selon l'une des revendications 1 à 5, **caractérisée en ce que** la partie de rotor (1) présente un enroulement court-circuité ou un rotor à cage.

7. Pompe rotative selon l'une des revendications 1 à 6, **caractérisée en ce que** la partie de rotor (1) est entourée par une enveloppe de rotor (1a), réalisée en un matériau non ferromagnétique, notamment en matière synthétique, métal, céramique ou en un matériau biocompatible.

8. Pompe rotative selon l'une des revendications 1 à 7, **caractérisée en ce que** la partie de rotor (1) ainsi que le stator (7) sont réalisés mutuellement de façon et que les enroulements électriques (8a, 8b, 8c, 8d, 8e, 8f, 8g, 8h) peuvent être commandés par un dispositif de commande (40) de façon que la partie de rotor (1) du moteur sans palier (4) puisse être entraînée selon le principe de fonctionnement d'un moteur à réluctance, d'un moteur synchrone ou d'un moteur à induction.

9. Pompe rotative selon la revendication 8, **caractérisée en ce que** le stator (7) présente un enroulement d'entraînement (WA) avec un nombre de paires de pôles p et un enroulement de commande (WS) avec un nombre de paires de pôles p+1 ou p-1.

10. Pompe rotative selon l'une des revendications 1 à 9, **caractérisée en ce qu'**il est disposé dans la direction axiale (A) à côté du rotor (2) au moins sur un côté un emplacement de palier de secours (9b).

11. Pompe rotative selon l'une des revendications 1 à 10, **caractérisée en ce que** le rotor (2) et un tronçon partiel de la paroi intérieure du boîtier (9) sont réalisés pour s'étendre mutuellement de façon à former un palier hydrodynamique agissant au moins dans la direction axiale (A).

12. Pompe rotative selon l'une des revendications 1 à 11, **caractérisée en ce que** le rotor (2) présente à la surface orientée vers le boîtier (9) au moins une rainure hélicoïdale pour réaliser une crapaudine.

13. Pompe rotative selon l'une des revendications 1 à 12, **caractérisée en ce que** la pompe rotative (3) est réalisée comme pompe axiale (3a), et **en ce que** la partie de rotor (1) présente dans le centre de rotation un évidement dans lequel sont disposées les aubes (1b) du rotor (2) réalisé comme roue axiale (2a).

14. Pompe rotative selon la revendication 13, **caractérisée en ce que** les aubes (1b) sont disposées pour qu'elles fassent saillie dans la direction axiale (A) sur l'enveloppe de rotor (1a).

15. Pompe rotative selon l'une des revendications 1 à 12, **caractérisée en ce que** la pompe rotative (3) est réalisée comme pompe axiale (3a), **en ce que** le rotor (2) est constitué seulement de la partie de rotor (1), et **en ce que** la partie de rotor (1) est réalisée pour s'étendre de façon que celle-ci forme en même temps les aubes (1b) de la roue axiale (2a).

16. Pompe rotative selon l'une des revendications 1 à 12, **caractérisée en ce que** la pompe rotative (3) est réalisée comme pompe centrifuge (3b), et **en ce que** le rotor (2) est réalisé comme roue centrifuge (2b) et présente dans la direction axiale (A) au moins sur un côté des aubes (1b).

17. Pompe rotative selon la revendication 16, **caractérisée en ce que** la paroi intérieure du boîtier (9) et/ou la roue centrifuge (2b) présentent des éléments partiels (100a, 100b, 104, 105, 103f, 1l, 1k) agissant sur l'écoulement pour provoquer pendant un fonctionnement de pompe une force agissant sensiblement dans la direction axiale (A) sur la roue centrifuge (2b).

18. Pompe rotative selon l'une des revendications 16 ou 17, **caractérisée en ce que** le boîtier (9) avec la roue centrifuge (2b) située à l'intérieur est réalisé pour pouvoir être inséré au moins partiellement dans le stator (7) et retiré de nouveau de celui-ci.

19. Pompe rotative selon l'une des revendications 1 à 18, **caractérisée en ce que** la partie de rotor (1) comprend au moins un aimant permanent annulaire (80b, 80c), et **en ce que** la pompe rotative (3) comprend au moins un autre aimant permanent fixe (80a, 80b), et **en ce que** les aimants permanents (80b, 80c, 80a, 80d) sont polarisés et disposés pour agir mutuellement de façon à produire une force de répulsion, agissant dans la direction axiale (A), sur la partie de rotor (1).

20. Pompe de sang comportant une pompe rotative (3, 3a, 3b) selon l'une des revendications 1 à 19.
